# EUROPEAN PATENT SPECIFICATION

(11) **EP 3 148 435 B1**
(45) Date of publication and mention of the grant of the patent: **18.10.2023**
(21) Application number: 15838411.5
(22) Date of filing: 04.09.2015
(51) Int. Cl.: A61B 5/11, A61J 7/04, A61B 5/0205, A61B 7/04, A61F 2/76, G02C 11/00, G06F 21/35, G07C 9/00, G08B 21/02, A61B 5/00

(54) **SYSTEM FOR MONITORING HEALTH RELATED INFORMATION FOR INDIVIDUALS**
SYSTEM ZUR ÜBERWACHUNG VON INFORMATIONEN IM ZUSAMMENHANG MIT GESUNDHEIT FÜR PERSONEN
SYSTÈME PERMETTANT DE SURVEILLER DES INFORMATIONS LIÉES À LA SANTÉ POUR DES INDIVIDUS

(30) Priority: 05.09.2014 US 201462046406 P; 21.11.2014 US 201414550406; 05.12.2014 US 201414562454; 30.01.2015 US 201514610589
(43) Date of publication of application: 05.04.2017
(73) Proprietor: Beijing Zitiao Network Technology Co., Ltd., Beijing 100190 (CN)
(72) Inventor: SALES, Jay William, Sacramento, CA 95814 (US); KLOSINSKI, JR. Richard Chester, Sacramento, CA 95814 (US); WORKMAN, Matthew Allen, Sacramento, CA 95814 (US); MURPHY, Meghan Kathleen, Sacramento, CA 95814 (US); STEEN, Matthew David, Sacramento, CA 95814 (US)
(74) Representative: Black, Diego
(86) International application number: PCT/US2015/048612
(87) International publication number: WO 2016/037091

(56) References cited:
- WO-A1-2007/088374
- WO-A1-2008/073806
- WO-A1-2016/029803
- US-A1- 2001 031 031
- US-A1- 2007 112 287
- US-A1- 2012 206 485
- US-A1- 2013 024 022
- US-A1- 2014 063 242

## Description

### CROSS-REFERENCE TO RELATED APPLICATIONS

This application claims the benefit of U.S. Patent Application No. 14/610,589, filed January 30, 2015, entitled "Systems and Method for Monitoring an Individual's Compliance with a Weight Loss Plan," U.S. Patent Application No. 14/550,406, filed November 21, 2014, entitled "Wearable Gait Monitoring Apparatus, Systems, and Related Methods," and U.S. Patent Application No. 14/562,454, filed December 5, 2014, entitled "System for Monitoring Individuals as They Age in Place," which all claim the benefit of U.S. Provisional Patent Application No. 62/046,406, filed September 5, 2014, entitled, "Wearable Health Computer Apparatus, Systems, and Related Methods,".

### BACKGROUND

Being able to monitor elderly individuals who live independently at home has become increasingly important due, in part, to the high cost of elder care facilities. Accordingly, there is a need for improved systems and methods for monitoring the activities and wellbeing of elderly individuals living at home. There is a similar need for monitoring the activities and wellbeing of individuals with special needs living outside of an institutional setting.

Furthermore, observing a person's gait is often an important clinical step in diagnosing certain types of musculoskeletal and neurological conditions. Proper gait diagnosis may also be valuable in properly fitting a patient with a prosthesis. Currently, gait analysis is largely dependent on the subjective perception of a trained professional. Such manual diagnosis methods may rely on a small set of observable activities and may have inherent inaccuracies, creating the potential for misdiagnosis and the delay of proper treatment. Thus, there is currently a need for improved systems and methods for diagnosing the gait of an individual.

Moreover, public health officials concur that Americans are not adhering to current healthful lifestyle recommendations. Only one in five of the US population follows recommendations for fruit and vegetable consumption. Only one in four adheres to the recommendations for exercise, and three of four adhere to recommendations not to smoke. Possibly, the most telling statistic is that only three of every 100 US adults follow all recommendations to consume five fruits and vegetables daily, get regular physical activity, maintain a healthy weight, and not smoke. Being able to monitor an individual's compliance with weight loss recommendations is advantageous for the individual, for health care providers and for insurance companies. Accordingly, there is a need for improved systems and methods for monitoring an individual's compliance with a weight loss regime.

Various embodiments of the present systems and methods recognize and address the foregoing considerations, and others, of prior art systems and methods, such as disclosed in document WO2007/08837.

### SUMMARY OF THE VARIOUS EMBODIMENTS

The invention is defined by the appended claims. It is noted that the disclosed methods are not part of the invention.

### BRIEF DESCRIPTION OF THE DRAWINGS

Various embodiments of systems and methods for assessing a user's activities and movements are described below. In the course of this description, reference will be made to the accompanying drawings, which are not necessarily drawn to scale and wherein:
Figure 1A is a block diagram of a Behavior Pattern Analysis System in accordance with an embodiment of the present system;
Figure 1B is a block diagram of an exemplary system for monitoring an individual's gait in accordance with an embodiment of the present system;
Figure 1C is a block diagram of a weight loss compliance system in accordance with an embodiment of the present system;
Figure 1D is a block diagram of a wearable health monitoring system in accordance with an embodiment of the present system;
Figure 2 is a block diagram of the computer for use in any one of Figures 1A - 1C;
Figure 3 is a perspective view of computerized eyewear according to a particular embodiment for use with the systems shown in Figures 1A - 1C;
Figure 4A is a flowchart that generally illustrates various steps executed by a Behavior Pattern Analysis Module according to a particular embodiment;
Figure 4B depicts a flowchart that generally illustrates a method of monitoring an individual's gait;
Figure 4C illustrates a flowchart that generally illustrates various steps executed by a Weight Loss Compliance Module according to a particular embodiment;
Figure 5 depicts a flowchart that generally illustrates various steps executed by a Health Data Acquisition Module according to a particular embodiment;
Figures 6A-6C depict a flowchart that generally illustrates various steps executed by an Actionable Data Collection Module according to a particular embodiment;
Figure 7 depicts a flowchart that generally illustrates various steps executed by a Decision Making Data Acquisition Module according to a particular embodiment; and
Figure 8 depicts a flowchart that generally illustrates various steps executed by a Processing and Reporting Module according to a particular embodiment.

### DETAILED DESCRIPTION OF SOME EMBODIMENTS

Various embodiments will now be described more fully hereinafter with reference to the accompanying drawings. It should be understood that the invention may be embodied in many different forms and should not be construed as limited to the embodiments set forth herein. Rather, these embodiments are provided so that this disclosure will be thorough and complete, and will fully convey the scope of the invention as defined by the claims to those skilled in the art. Like numbers refer to like elements throughout.

### Behavior Pattern Analysis System Overview

A wearable health monitoring system, according to various embodiments, may include a suitable wearable device that is configured to monitor one or more movements, activities, and/or health attributes of a wearer (e.g., user). Suitable wearable devices may include, for example: (1) pair of eyewear (e.g., goggles or eyeglasses); (2) one or more contact lenses; (3) a wristwatch; (4) an article of clothing (e.g., such as a suitable shirt, pair of pants, undergarment, compression sleeve, etc.); (5) footwear; (6) a hat; (7) a helmet; (8) an adhesive strip or other tag that may be selectively attached to an individual or the individual's clothing; (9) a computing device that is embedded into a portion of an individual's body (e.g., under the individual's skin, or within a medical device, such as a pacemaker); (10) an orthopedic cast, or (11) any other suitable wearable item. In a particular example, a wearable health monitoring system embodied as a pair of eyewear may enable the system to monitor what an individual is sensing (e.g., touching, seeing, hearing, smelling, and/or tasting) based at least in part on a proximity of the eyewear to the wearer's sensory systems (e.g., skin, eyes, mouth, ears, nose) when worn by the wearer.

In various embodiments, the system comprises one or more sensors that are configured to determine one or more current physical attributes of the wearer (e.g., heart rate, brain wave activity, movement, body temperature, blood pressure, oxygen saturation level, etc...). The one or more sensors may include, for example: (1) one or more heart rate monitors; (2) one or more electrocardiograms (EKG); (3), one or more electroencephalograms (EEG); (4) one or more pedometers; (5) one or more thermometers; (6) one or more transdermal transmitter sensors; (7) one or more front-facing cameras; (8) one or more eye-facing cameras; (9) one or more microphones; (10) one or more accelerometers; (11) one or more blood pressure sensors; (12) one or more pulse oximeters; (13) one or more respiratory rate sensors; (14) one or more blood alcohol concentration (BAC) sensors; (15) one or more near-field communication sensors; (16) one or more motion sensors; (17) one or more gyroscopes; (18) one or more geomagnetic sensors; (19) one or more global positioning system sensors; (20) one or more impact sensors; and/or (21) any other suitable one or more sensors.

In particular embodiments, the system is configured to gather data, for example, using the one or more sensors, about the wearer (e.g., such as the wearer's body temperature, balance, heart rate, level of physical activity, diet (e.g., food recently eaten), compliance with a prescribed medical regimen (e.g., medications recently taken), position, movements (e.g., body movements, facial muscle movements), location, distance traveled, etc.). In various embodiments, the system is configured to, for example: (1) store the gathered data associated with the user; (2) provide the data to one or more medical professionals, for example, to aid in the diagnosis and/or treatment of the user; (3) use the data to predict one or more medical issues with the user (e.g., the illness or death of the user); and/or (4) take any other suitable action based at least in part on the gathered data.

In a particular implementation, the system's wearable device is a pair of computerized eyewear that comprises one or more sensors for monitoring one or more day-to-day activities of an elderly individual as they "age in place" (e.g., they live in a non-institutional setting). In particular embodiments, the one or more sensors are coupled to (e.g., connected to, embedded in, etc.) the pair of glasses, which may be, for example, a pair of computerized or non-computerized eyeglasses. In particular embodiments, the individual is a senior citizen who lives at least substantially independently.

In particular embodiments, the wearable computing device comprises one or more location sensors (e.g., geomagnetic sensors, etc.), motion sensors (e.g., accelerometers, gyroscopes, magnetic sensors, pressure sensors, etc.), and/or impact sensors that are adapted to sense the movement and location of the individual. In various embodiments, the wearable device is adapted to facilitate the transmission of this movement information to a remote computing device (e.g., a handheld computing device, an automated dialing device, a central server, or any other suitable smart device that may, in various embodiments, contain a wireless communications device that can connect to the wearable computing device) that analyzes the information to determine whether the individual's movement patterns are consistent with the individual's typical (e.g., past) movement patterns. If the movement patterns are inconsistent with the individual's typical movement patterns, the system may, for example, generate and transmit an alert to a third party (e.g., a physician, relative of the individual, other caretaker, police, etc.) informing the third party of the irregularities in the individual's movement. The third party may then, for example, check on the individual to make sure that the individual does not require assistance.

In further embodiments, the wearable device may be adapted, for example, to monitor: (1) an individual's compliance with a prescribed treatment plan (e.g., compliance with a medication schedule); (2) an individual's compliance with a diet; and/or (3) whether an individual leaves a prescribed area defined by a geo-fence (e.g., a virtual fence). The system may do this, for example, by using any suitable sensors (e.g., location sensors, cameras, etc...) associated with the wearable device.

### Wearable Gait Monitoring System Overview

A system, according to various embodiments, includes eyewear (or any other suitable wearable device) that includes one or more sensors (e.g., one or more heart rate monitors, one or more electrocardiograms (EKG), one or more electroencephalograms (EEG), one or more pedometers, one or more thermometers, one or more transdermal sensors, one or more front-facing cameras, one or more eye-facing cameras, one or more microphones, one or more accelerometers, one or more blood pressure sensors, one or more pulse oximeters, one or more respiratory rate sensors, one or more blood alcohol concentration (BAC) sensors, one or more motion sensors, one or more gyroscopes, one or more geomagnetic sensors, one or more global positioning system sensors, one or more impact sensors, or any other suitable one or more sensors) that may be used to monitor the gait of an individual. The system may further include one or more suitable computing devices for analyzing the individual's gait. This information may then be used, for example, to: (1) identify one or more medical conditions associated with the individual; (2) assess the fit of a prosthetic device worn by the individual, and/or (3) assess an individual's recovery from a particular injury or medical procedure.

### System for Monitoring an Individual's Compliance with a Weight Loss Plan Overview

A weight loss compliance system, according to various embodiments, may include a suitable wearable device that is configured to monitor one or more of a wearer's (e.g., a user) food consumption, physical activities, sleep patterns, and/or compliance with a medicine regime (e.g., weight loss medicine/supplement). In particular embodiments, the system is configured to gather data, for example, using one or more sensors, about the wearer (e.g., such as the wearer's body temperature, heart rate, level of physical activity, geographic location, distance traveled, diet (e.g., food consumed, calories for each meal, total calories, etc.), compliance with a prescribed medical regimen (e.g., medications recently taken, dose, side effects), sleeping patterns (e.g., hours slept, type of sleep, quality of sleep), etc.). In various embodiments, the system is configured to, for example: (1) identify a food and quantity of the food that the wearer is preparing to ingest; (2) track the identity and the quantity of food (e.g., the nutritional value, the calories associated with the food, the date and time the food is ingested, etc.); (3) compare the identity and quantity of the food to a predetermined weight loss plan (e.g., a dietary plan); (4) calculate one or more recommendations to assist the wearer in complying with the predetermined weight loss plan; and/or (5) notify the wearer of the one or more recommendations.

In particular embodiments, the wearable device is adapted to facilitate the transmission of the data captured by the one or more sensors to a remote computing device (e.g., a handheld computing device, a central server, or any other suitable smart device that may, in various embodiments, contain a wireless communications device that can connect to the wearable computing device) that analyzes the information to determine whether the wearer is complying with the predetermined weight loss plan (e.g., a dietary plan, an exercise plan, a medicine plan, a sleep plan, or a combination of one or more of the proceeding). As the system tracks the wearer's food intake (e.g., calories, nutritional values, etc.), physical activities, sleep patterns, and/or medicine intake, the system may provide the wearer feedback, warnings, and/or recommendations on (1) how to better comply with the weight plan (e.g., eat more green vegetables or fruit, don't eat fast food, you should eat smaller portions, etc.), (2) how a particular food or quantity will impact the weight loss plan (e.g., if you eat this, you must perform a particular physical activity to offset the calories, etc.), and/or (3) how a medicine can impact the wearer's ability to lose weight (e.g., a particular side effect of the medicine is weight gain/loss, etc.).

Suitable wearable devices may include, for example: (1) a pair of eyewear (e.g., goggles or eyeglasses); (2) one or more contact lenses; (3) a wristwatch; (4) an article of clothing (e.g., such as a suitable shirt, pair of pants, undergarment, compression sleeve, etc.); (5) footwear; (6) a hat; (7) a helmet; (8) an adhesive strip or other tag that may be selectively attached to an individual or the individual's clothing; (9) a computing device that is embedded into a portion of an individual's body (e.g., under the individual's skin, or within a medical device, such as a pacemaker); (10) an orthopedic cast; or (11) any other suitable wearable item.

In various embodiments, the wearable device comprises one or more sensors that are configured to sense the wearer's food intake, physical activities, sleep patterns, and medicine intake (e.g., prescription weight loss drugs, over-the-counter weight loss drugs, nutritional supplements, prescription drugs, etc.). The one or more sensors may include, for example: (1) one or more forward facing cameras; (2) one or more global positioning sensors; (3), one or more olfactory sensors; (4) one or more pedometers; (5) one or more microphones; (6) one or more accelerometers; (7) one or more blood pressure sensors; (8) one or more pulse oximeters; (9) one or more respiratory rate sensors; (10) one or more near-field communication chips; (11) one or more gyroscopes; (12) one or more geomagnetic sensors; (13) one or more global positioning system chips; and/or (14) any other suitable one or more sensors.

In a particular implementation, the system's wearable device includes a pair of computerized eyewear that comprises the one or more sensors for monitoring the wearer's compliance with a weight loss plan. In particular embodiments, the one or more sensors are coupled to (e.g., connected to, embedded in, etc.) the pair of glasses, which may be, for example, a pair of computerized or non-computerized eyeglasses. In particular embodiments, the wearer is a person trying to lose weight, become more physically active, under orders of a doctor to lose weight for medical reasons or any other person trying to live a more active and healthy life.

### Health Monitoring System Overview

A wearable health monitoring system, in various embodiments, may, for example, be embodied in any suitable wearable device configured to monitor one or more health attributes of a wearer. The system may, for example, be embodied as a pair of eyewear, as contact lenses, as a wristwatch, as a suitable piece of clothing (e.g., such as a suitable shirt, pair of pants, undergarment, compression sleeve, etc.), as footwear, as a hat, as an orthopedic cast, or any other suitable wearable item. In a particular example, a wearable health monitoring system embodied as a pair of eyewear may enable the system to access all five of a user's senses (e.g., touch, sight, sound, smell, and taste) based at least in part on a proximity of the eyewear to the user's sensory systems (e.g., eyes, mouth, ears, nose) when worn by the user.

In various embodiments, the system comprises one or more sensors configured to determine one or more attributes of the wearer. The one or more sensors may include, for example, one or more heart rate monitors, one or more pedometers, one or more thermometers, one or more cameras, one or more microphones, one or more accelerometers, one or more blood pressure sensors or any other suitable one or more sensors. In particular embodiments, the system is configured to gather data, for example, using the one or more sensors, about the user (e.g., such as temperature, balance, heart rate, activity, activity levels, food eaten, medications taken, steps taken, position, etc.). In various embodiments, the system is configured to, for example: (1) store the gathered data associated with the user; (2) provide the data to one or more medical professionals, for example, to aid in the diagnosis and/or treatment of the user; (3) use the data to predict one or more medical issues with the user; and/or (4) take any other suitable action based at least in part on the gathered data.

### Exemplary Technical Platforms

As will be appreciated by one skilled in the relevant field, the present systems and methods may be, for example, embodied as a computer system, a method, or a computer program product. Accordingly, various embodiments may be entirely hardware or a combination of hardware and software. Furthermore, particular embodiments may take the form of a computer program product stored on a computer-readable storage medium having computer-readable instructions (e.g., software) embodied in the storage medium. Various embodiments may also take the form of Internet-implemented computer software. Any suitable computer-readable storage medium may be utilized including, for example, hard disks, compact disks, DVDs, optical storage devices, and/or magnetic storage devices.

Various embodiments are described below with reference to block diagram and flowchart illustrations of methods, apparatuses, (e.g., systems), and computer program products. It should be understood that each block of the block diagrams and flowchart illustrations, and combinations of blocks in the block diagrams and flowchart illustrations, respectively, can be implemented by a computer executing computer program instructions. These computer program instructions may be loaded onto a general purpose computer, a special purpose computer, or other programmable data processing apparatus that can direct a computer or other programmable data processing apparatus to function in a particular manner such that the instructions stored in the computer-readable memory produce an article of manufacture that is configured for implementing the functions specified in the flowchart block or blocks.

The computer instructions may execute entirely on the user's computer, partly on the user's computer, as a stand-alone software package, partly on a user's computer and partly on a remote computer, or entirely on the remote computer or server. In the latter scenario, the remote computer may be connected to the user's computer through any type of network, including but not limited to: (1) a local area network (LAN); (2) a wide area network (WAN); (3) a cellular network; or (4) the connection may be made to an external computer (for example, through the Internet using an Internet Service Provider).

These computer program instructions may also be stored in a computer-readable memory that can direct a computer or other programmable data processing apparatus to function in a particular manner such that the instructions stored in the computer-readable memory produce an article of manufacture that is configured for implementing the function specified in the flowchart block or blocks. The computer program instructions may also be loaded onto a computer or other programmable data processing apparatus to cause a series of operational steps to be performed on the computer or other programmable apparatus to produce a computer-implemented process (e.g., method) such that the instructions that execute on the computer or other programmable apparatus provide steps for implementing the functions specified in the flowchart block or blocks.

### Example System Architecture - Behavior Pattern Analysis System

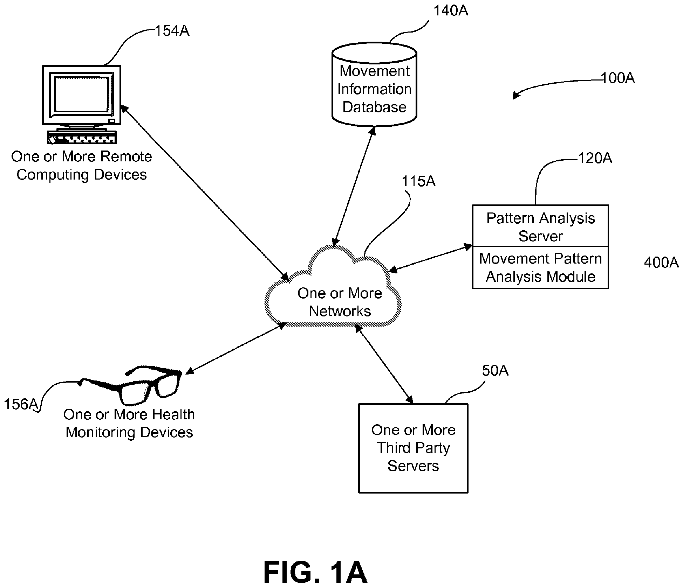

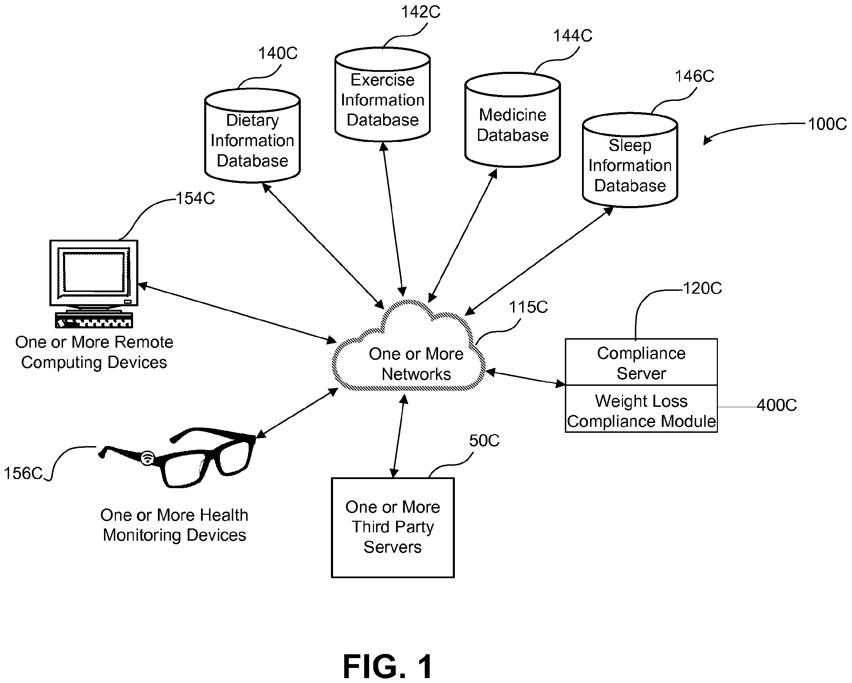

Figure 1A is a block diagram of a behavior pattern analysis system 100A according to particular embodiments. As may be understood from this figure, the behavior pattern analysis system 100A includes one or more networks 115A, one or more third party servers 50A, a pattern analysis server 120A that includes a behavior pattern analysis module 400A, a movement information database 140A, one or more remote computing devices 154A (e.g., such as a smart phone, a tablet computer, a wearable computing device, a laptop computer, a desktop computer, a Bluetooth device, an automated dialing apparatus, etc.), and one or more wearable health monitoring devices 156A, which may, for example, be embodied as one or more of eyewear, headwear, clothing, a watch, a hat, a helmet, a cast, an adhesive bandage, a piece of jewelry (e.g., a ring, earring, necklace, bracelet, etc.), or any other suitable wearable device. In particular embodiments, the one or more computer networks 115A facilitate communication between the one or more third party servers 50A, the pattern analysis server 120A, the movement information database 140A, the one or more remote computing devices 154A, and the one or more health monitoring devices 156A.

The one or more networks 115A may include any of a variety of types of wired or wireless computer networks such as the Internet, a private intranet, a mesh network, a public switch telephone network (PSTN), or any other type of network (e.g., a network that uses Bluetooth or near field communications to facilitate communication between computing devices). The communication link between the one or more remote computing devices 154A and the pattern analysis server 120A may be, for example, implemented via a local area network (LAN) or via the Internet.

### Example System Architecture - Wearable Gait Monitoring System

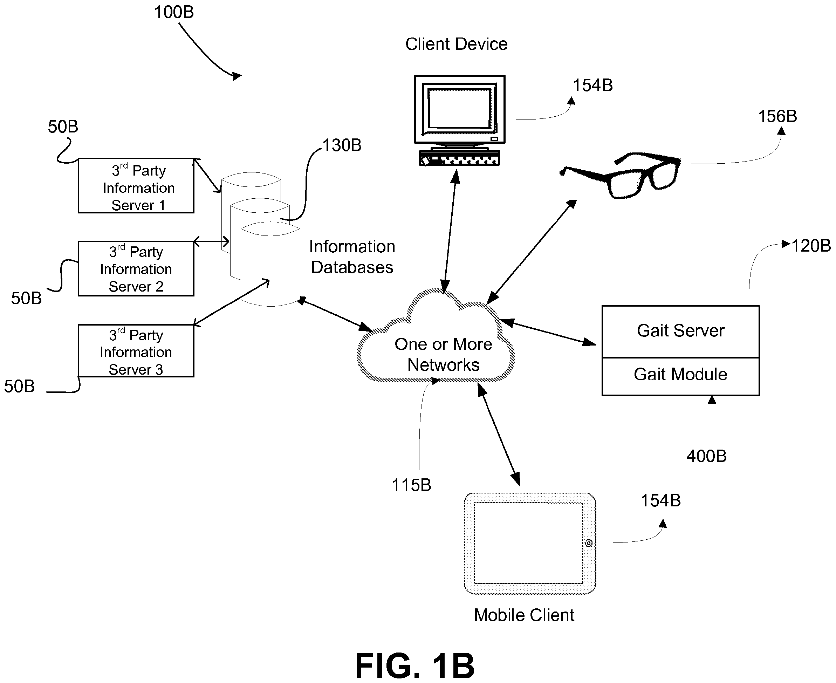

Figure 1B is a block diagram of a wearable gait monitoring system 100B according to a particular embodiment. As may be understood from this figure, the wearable gait monitoring system 100 includes one or more computer networks 115B, one or more third party servers 50B, 50B, 50B, a gait server 120B, a database 130B, one or more remote computing devices 110B (e.g., such as a smart phone, a tablet computer, a wearable computing device, a laptop computer, etc.), and one or more wearable gait monitoring device(s) 156B, which may, for example, be embodied as eyewear (e.g., glasses or goggles), clothing, a watch, a hat, a cast, an adhesive bandage, a piece of jewelry (e.g., a ring, earring, necklace, etc.), and/or any other suitable wearable device.

In various embodiments, the one or more wearable gait monitoring device(s) 156B may further comprise at least one processor and one or more sensors (e.g., an accelerometer, a magnetometer, a gyroscope, a front-facing camera, a location sensor such as a GPS unit, etc.). In particular embodiments, the system is configured to gather data, for example, using the one or more sensors, regarding the user's gait as the user walks or runs (e.g., the user's stride cadence, the user's speed (e.g., the speed of the user's feet and/or body), the orientation of the user (e.g., the orientation of the user's body and/or feet), the elevation of the user's respective feet from the ground, the movement of the user's head such as bobbing, etc.).

In various embodiments, the database is configured to store information regarding gait patterns associated with various predetermined medical conditions. The system is configured to store information regarding normal gait patterns for a particular individual or individuals who are similar in physical stature to the particular individual. In various embodiments, the database stores past information regarding an individual's gait and may include recent gait measurements for the individual, which may, for example, be used to track the individual's progress in improving their gait (e.g., after an injury or a medical procedure).

The one or more computer networks 115B may include any of a variety of types of wired or wireless computer networks such as the Internet, a private intranet, a mesh network, a public switch telephone network (PSTN), or any other type of network (e.g., a network that uses Bluetooth or near field communications to facilitate communication between computers). The communication link between the wearable gait monitoring system 100B and the database 130B may be, for example, implemented via a local area network (LAN) or via the Internet. In particular embodiments, the one or more computer networks 115b facilitate communication between the one or more third party servers 50b, 50b, 50b, the gait server 120b, the database 130b, and one or more remote computing devices 110b. In various embodiments, the handheld device 110a is configured to communicate with the wearable gait monitoring device 156B via, for example, Bluetooth. In various other embodiments, the wearable gait monitoring device 156B may communicate with a remote server, for example, the gait server 120B, via a cellular communication or wireless Internet connection. In yet other embodiments, the system may be further configured to allow the wearable gait monitoring device 156B to communicate with the remote server (e.g., the gait server 120B), without the intermediary handheld device 110B.

### Example System Architecture - Systems for Monitoring Compliance with a Weight Loss Plan

Figure 1C is a block diagram of a weight loss compliance system 100C according to particular embodiments. As may be understood from this figure, the weight loss compliance system 100C includes one or more networks 115C, one or more third party servers 50C, a compliance server 120C that includes a weight loss compliance module 400C, a dietary information database 140C, an exercise information database 142C, a medicine database 144C, a sleep information database 146C, one or more remote computing devices 154C (e.g., such as a smart phone, a tablet computer, a wearable computing device, a laptop computer, a desktop computer, a Bluetooth device, an automated dialing apparatus, etc.), and one or more health monitoring devices 156C, which may, for example, be embodied as one or more of eyewear, headwear, clothing, a watch, a hat, a helmet, a cast, an adhesive bandage, a piece of jewelry (e.g., a ring, earring, necklace, bracelet, etc.), or any other suitable wearable device. In particular embodiments, the one or more computer networks 115C facilitate communication between the one or more third party servers 50C, the compliance server 120C, the dietary information database 140C, the exercise information database 142C, the medicine database 144C, the sleep information database 146C, the one or more remote computing devices 154C, and/or the one or more health monitoring devices 156C.

The one or more networks 115C may include any of a variety of types of wired or wireless computer networks such as the Internet, a private intranet, a mesh network, a public switch telephone network (PSTN), or any other type of network (e.g., a network that uses Bluetooth or near field communications to facilitate communication between computing devices). The communication link between the one or more remote computing devices 154C and the compliance server 120C may be, for example, implemented via a Local Area Network (LAN) or via the Internet.

### Example System Architecture - Health Monitoring System

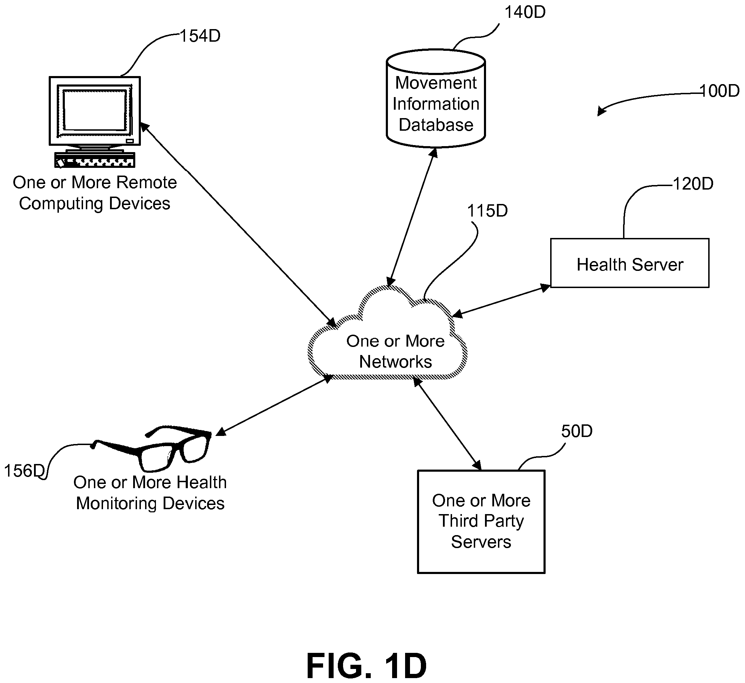

Figure 1D is a block diagram of a wearable health monitor system 100D according to a particular embodiment. As may be understood from this figure, the wearable health monitor system 100D includes one or more computer networks 115D, one or more third party servers 50, a health server 120D, a database 140D, one or more remote computing devices 154D (e.g., such as a smart phone, a tablet computer, a wearable computing device, a laptop computer, etc.), and one or more wearable health monitoring devices 156D, which may, for example, be embodied as eyewear, clothing, a watch, a hat, a cast, an adhesive bandage, a piece of jewelry (e.g., a ring, earring, necklace, etc.), or any other suitable wearable device. In various embodiments, the one or more wearable health monitoring devices 156D comprise at least one processor and one or more sensors. In particular embodiments, the one or more computer networks facilitate communication between the one or more third party servers 50D, health server 120D, database 140D, and one or more remote computing devices 154D.

The one or more computer networks 115D may include any of a variety of types of wired or wireless computer networks such as the Internet, a private intranet, a mesh network, a public switch telephone network (PSTN), or any other type of network (e.g., a network that uses Bluetooth or near field communications to facilitate communication between computers). The communication link between the system information server 120D and the database 140D may be, for example, implemented via a Local Area Network (LAN) or via the Internet.

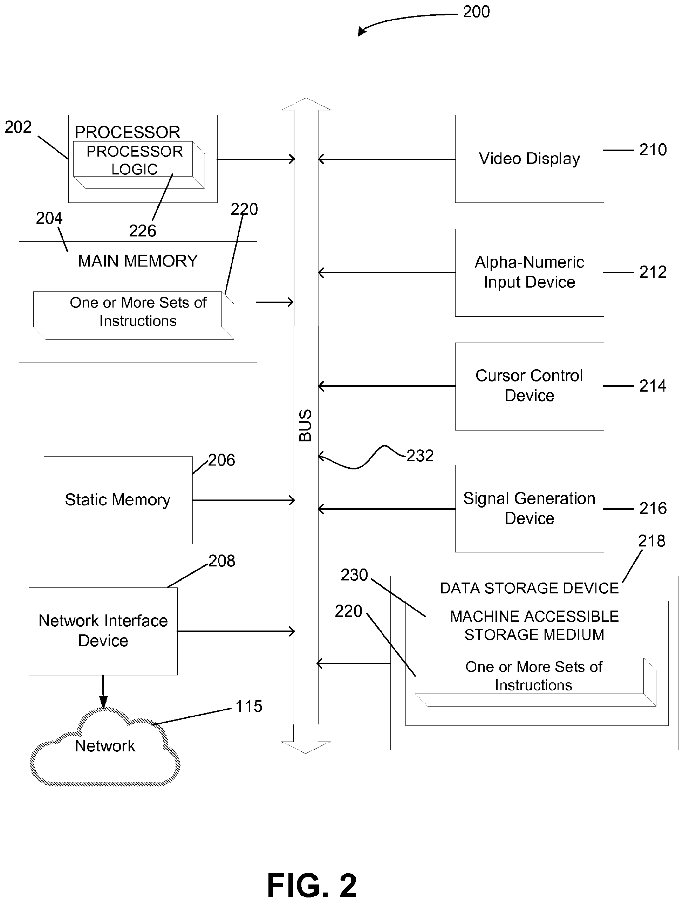

Figure 2 illustrates a diagrammatic representation of a computer architecture 200 for one or more of the pattern analysis server 120A, the gait server 120B, the compliance server 120C or the health server 120D that may be used respectively within the behavior pattern analysis system 100A, the wearable gait monitoring system 100B, the weight loss compliance system 100C or the health monitoring system 100D. It should be understood that the computer architecture 200 shown in Figure 2 may also represent the computer architecture for any one of the one or more remote computing devices 154A, 154B, 154C or 154D, one or more third party servers 50A, 50B, 50C or 50D and the one or more health monitoring devices 150A, 150B, 150C or 150D shown in Figures 1A, 1B, 1C or 1D.

In particular embodiments, the computer 200 may be connected (e.g., networked) to other computing devices in a LAN, an intranet, an extranet, and/or the Internet as shown in Figures 1A - 1D. As noted above, the Computer 200 may operate in the capacity of a server or a client computing device in a client-server network environment, or as a peer computing device in a peer-to-peer (or distributed) network environment. The computer 200 may be a desktop personal computing device (PC), a tablet PC, a set-top box (STB), a Personal Digital Assistant (PDA), a cellular telephone, a web appliance, a network router, a switch or bridge, or any other computing device capable of executing a set of instructions (sequential or otherwise) that specify actions to be taken by that computing device. Further, while only a single computing device is illustrated, the term "computing device" shall also be interpreted to include any collection of computing devices that individually or jointly execute a set (or multiple sets) of instructions to perform any one or more of the methodologies discussed herein.

An exemplary computer 200 includes a processing device 202, a main memory 204 (e.g., read-only memory (ROM), flash memory, dynamic random access memory (DRAM) such as synchronous DRAM (SDRAM) or Rambus DRAM (RDRAM), etc.), a static memory 206 (e.g., flash memory, static random access memory (SRAM), etc.), and a data storage device 218, which communicate with each other via a bus 232.

The processing device 202 represents one or more general-purpose or specific processing devices such as a microprocessor, a central processing unit (CPU), or the like. More particularly, the processing device 202 may be a complex instruction set computing (CISC) microprocessor, reduced instruction set computing (RISC) microprocessor, very long instruction word (VLIW) microprocessor, or processor implementing other instruction sets, or processors implementing a combination of instruction sets. The processing device 202 may also be one or more special-purpose processing devices such as an application specific integrated circuit (ASIC), a field programmable gate array (FPGA), a digital signal processor (DSP), network processor, or the like. The processing device 202 may be configured to execute processing logic 226 for performing various operations and steps discussed herein.

The computer 200 may further include a network interface device 208. The computer 200 may also include a video display unit 210 (e.g., a liquid crystal display (LCD) or a cathode ray tube (CRT)), an alpha-numeric input device 212 (e.g., a keyboard), a cursor control device 214 (e.g., a mouse), and a signal generation device 216 (e.g., a speaker).

The data storage device 218 may include a non-transitory computing device-accessible storage medium 230 (also known as a non-transitory computing device-readable storage medium or a non-transitory computing device-readable medium) on which is stored one or more sets of instructions (e.g., the Movement Pattern Analysis Module 400A, the Gait Module 400B, the Weight Loss Compliance Module 400C, the Health Data Acquisition Module 500, the Actionable Data Collection Module 600, the Decision Making Data Acquisition Module 700 or the Processing and Reporting Module 800) embodying any one or more of the methodologies or functions described herein. The one or more sets of instructions may also reside, completely or at least partially, within the main memory 204 and/or within the processing device 202 during execution thereof by the computer 200 - the main memory 204 and the processing device 202 also constituting computing device-accessible storage media. The one or more sets of instructions may further be transmitted or received over a network 115A, 115B, 115C, or 115D via a network interface device 208.

While the computing device-accessible storage medium 230 is shown in an exemplary embodiment to be a single medium, the term "computing device-accessible storage medium" should be understood to include a single medium or multiple media (e.g., a centralized or distributed database, and/or associated caches and servers) that store the one or more sets of instructions. The term "computing device-accessible storage medium" should also be understood to include any medium that is capable of storing, encoding, or carrying a set of instructions for execution by the computing device and that causes the computing device to include any one or more of the methodologies of the present invention. The term "computing device-accessible storage medium" should accordingly be understood to include, but not be limited to, solid-state memories, optical and magnetic media, etc.

### Structure of the Eyewear

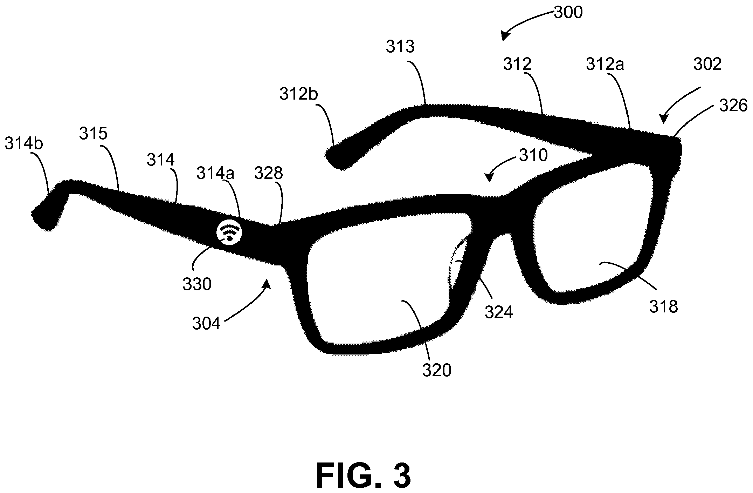

As shown in Figure 3, the one or more wearable health monitoring devices 156A, the one or more wearable gait monitoring device 156B, the one or more health monitoring devices 156C, or the one or more health monitoring device 156D may be configured as eyewear 300, which according to various embodiments, includes: (1) an eyewear frame 310; (2) a first temple 312; and (3) a second temple 314, as described below.

### Eyewear Frame

Referring still to Figure 3, eyewear 300, in various embodiments, includes any suitable eyewear frame 310 configured to support one or more lenses 318, 320. In the embodiment shown in this figure, the eyewear frame 310 has a first end 302 and a second end 304. The eyewear frame 310 may be made of any suitable material such as metal, ceramic, polymers or any combination thereof. In particular embodiments, the eyewear frame 310 is configured to support the first and second lenses 318, 320 about the full perimeter of the first and second lenses 318, 320. In other embodiments, the eyewear frame 310 may be configured to support the first and second lenses 318, 320 about only a portion of each respective lens. In various embodiments, the eyewear frame 310 is configured to support a number of lenses other than two lenses (e.g., a single lens, a plurality of lenses, etc.). In particular embodiments, the lenses 318, 320 may include prescription lenses, sunglass lenses, or any other suitable type of lens (e.g., reading lenses, non-prescription lenses), which may be formed from glass or polymers.

The eyewear frame 310 includes a first and second nose pad 322 (not shown in figures), 324, which may be configured to maintain the eyewear 300 adjacent the front of a wearer's face such that the lenses 318, 320 are positioned substantially in front of the wearer's eyes while the wearer is wearing the eyewear 300. In particular embodiments, the nose pads 322, 324 may comprise a material that is configured to be comfortable when worn by the wearer (e.g., rubber, etc.). In other embodiments, the nose pads may include any other suitable material (e.g., plastic, metal, etc.). In still other embodiments, the nose pads may be integrally formed with the frame 310.

The eyewear frame 310 includes a first and second hinge 326, 328 that attach the first and second temples 312, 314 to the frame first and second ends 302, 304, respectively. In various embodiments, the hinges may be formed by any suitable connection (e.g., tongue and groove, ball and socket, spring hinge, etc.). In particular embodiments, the first hinge 326 may be welded to, or integrally formed with, the frame 310 and the first temple 312 and the second hinge 328 may be welded to, or integrally formed with, the frame 310 and the second temple 314.

### First and Second Temples

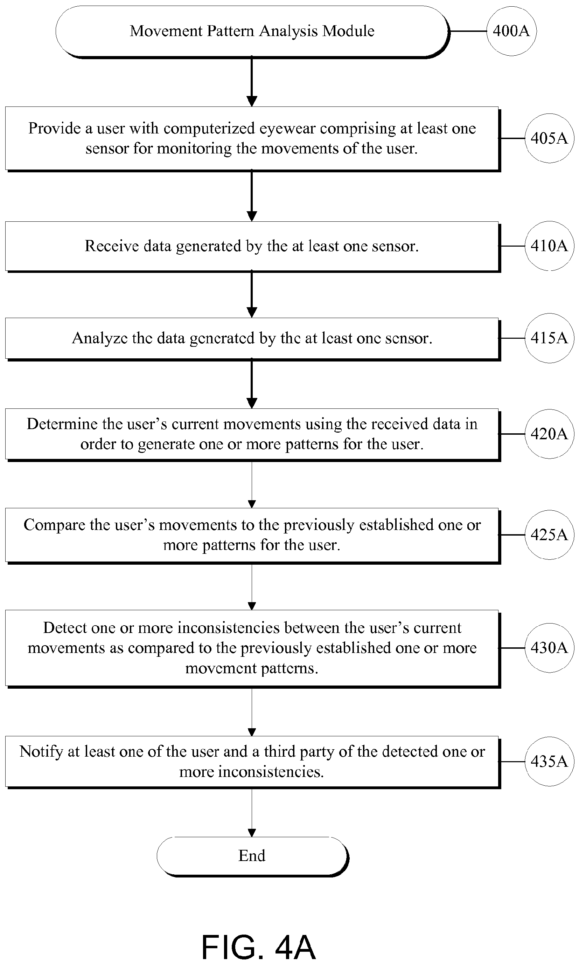

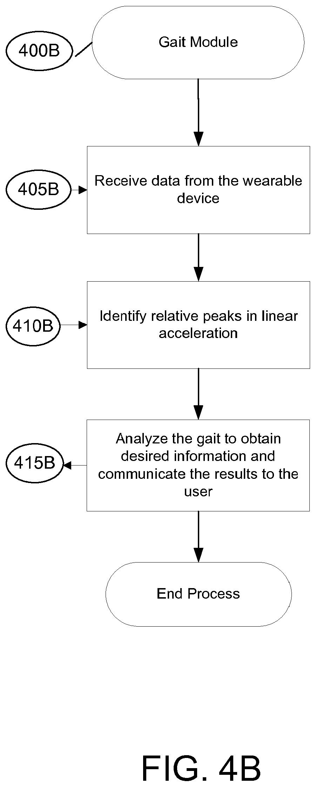

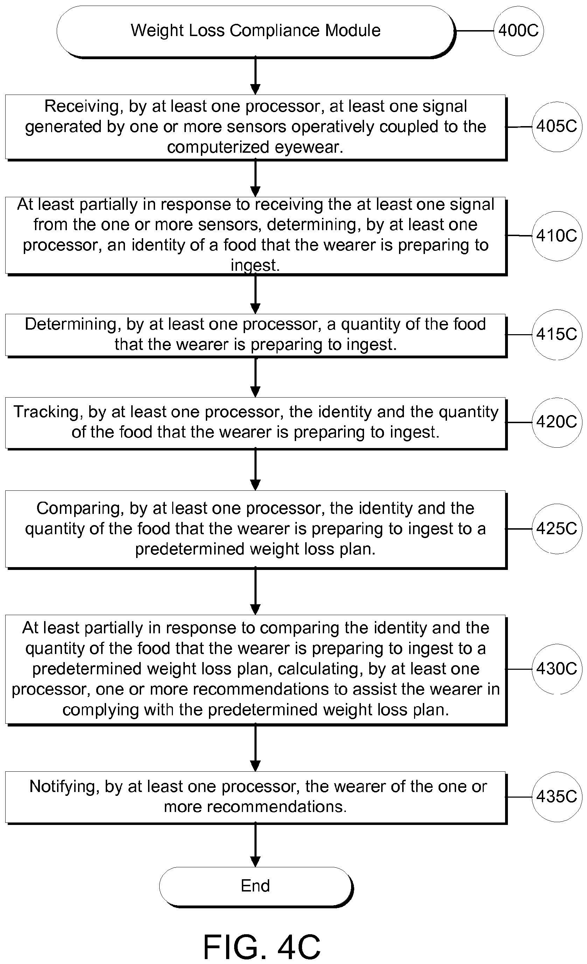

Still referring to Figure 4, the first temple 312, according to various embodiments, is rotatably connected to the frame 310 at a right angle to extend the first temple 312 substantially perpendicular, substantially parallel, or anywhere in between the right angle to the frame 310. The first temple 312 has a first and second end 312a, 312b. Proximate the first temple second end 312b, the first temple 312 includes an earpiece 313 configured to be supported by a wearer's ear. Similarly, the second temple 314, according to various embodiments, is rotatably connected to the frame 310 at a right angle to extend the second temple 314 substantially perpendicular, substantially parallel, or anywhere in between the right angle to the frame 310. The second temple 314 has a first and second end 314a, 314b. Proximate the second temple second end 314b, the second temple 314 includes an earpiece 315 configured to be supported by a wearer's ear.

### Sensors

In various embodiments, the second temple 314 has one or more sensors 330 connected to the second temple 314. In various embodiments, the one or more sensors 330 may be coupled to the frame 310, the first and second temples 312, 314, the first and second lenses 318, 310, the nose piece 324, or any other portion of the eyewear 300 in any suitable way. For instance, the one or more sensors 330 may be embedded into the eyewear 300, coupled to the eyewear 300, and/or operatively coupled to the eyewear 300. In various embodiments, the one or more sensors may be formed at any point along the eyewear 300. For instance, a fingerprint reader may be disposed adjacent the first temple of the eyewear 300. In various embodiments, the one or more sensors may be formed in any shape. In addition, the one or more sensors may be formed on the inner (back) surface of the frame 310, the first and second temples 312, 414, the first and second lenses 318, 310, or any other portion of the eyewear 300. In other embodiments, the one or more sensors may be formed on the outer (front) surface of the frame 310, the first and second temples 312, 314, the first and second lenses 318, 310, or any other portion of the eyewear 300.

In various embodiments, the one or more sensors 330 that are coupled to the eyewear (or other wearable device) are adapted to detect the identity of a food, the quantity of a food, the identity of a medicine, the dose of the medicine, one or more physical activities performed by the wearer, the length of the physical activity, etc. In various embodiments, the one or more sensors coupled to the eyewear or other health monitoring device may include, for example, one or more of the following: a near-field communication chip, a gyroscope, a Bluetooth chip, a GPS unit, an RFID tag (passive or active), a fingerprint reader, an iris reader, a retinal scanner, a voice recognition sensor, a forward facing camera, an olfactory sensor, a heart rate monitor, an electrocardiogram (EKG), an electroencephalogram (EEG), a pedometer, a thermometer, a microphone, an accelerometer, a magnetometer, a blood pressure sensor, a pulse oximeter, a skin conductance response sensor, a blood sensor, any suitable biometric reader or any other suitable sensor. In particular embodiments, the sensors coupled to the eyewear may include one or more electronic communications devices such as a near field communication chip, a Bluetooth chip, a forward facing camera, a microphone and a GPS unit.

In various embodiments, the one or more sensors are coupled to a computing device that is associated with (e.g., embedded within, attached to) the eyewear or other wearable device. In particular embodiments, the eyewear or other wearable device comprises at least one processor, computer memory, suitable wireless communications components (e.g., a Bluetooth chip) and a power supply for powering the wearable device and/or the various sensors. In some such embodiments, the one or more sensors may be coupled to a Bluetooth device that is configured to transmit the one or more signals to a handheld wireless device.

In particular embodiments, the system is configured to receive input from a user (e.g., a wearer of the eyewear) via one or more gestures, for example, using at least one of the sensors described above. In various embodiments, the system may, for example, be configured to: (1) identify a gesture performed by the user; and (2) at least partially in response to identifying the gesture, perform a function associated with the gesture. In particular embodiments, the system may be configured to perform a particular function in response to identifying a particular gesture, where the particular gesture is associated with the particular function. In particular embodiments, the system may be configured to enable the user to provide one or more gestures for performing a particular function. In such embodiments, the system may, for example: (1) receive a selection of a particular function from the user; (2) receive input of one or more gestures from the user; and (3) associate the particular function with the one or more gestures.

In various embodiments, the one or more gestures may include, for example: (1) one or more hand gestures (e.g., a thumbs up, a wave, two thumbs up, holding up any particular number of fingers, making one or more fists, performing a particular movement with one or more hands, etc.); (2) one or more head movements (e.g., shaking of the user's head, a nod, etc.); (3) one or more eye movements (e.g., looking in a particular direction for a particular period of time, a wink, blinking, blinking in a particular pattern, etc.); (4) one or more facial movements (e.g., a smile, a frown, sticking out of a tongue, etc.); and/or (5) any suitable combination of these or any other suitable gestures.

In particular embodiments, the system is configured to identify the one or more gestures, for example, using a suitable imaging device (e.g., camera) that is part of the system. In particular embodiments, the imaging device may be directed toward an area in front of the user while the user is wearing the eyewear 300 and configured to identify gestures performed by the user's hands, arms, feet, legs, etc. In other embodiments, the system may include an imaging device directed toward the user's face and/or eyes while the user is wearing the eyewear 300 that is configured to identify gestures performed by the user's face and/or eyes.

In other embodiments, the system comprises one or more gyroscopes and/or accelerometers configured to determine a position or change in position of the eyewear 300 while the user is wearing the eyewear. In such embodiments, the one or more gyroscopes and/or accelerometers are configured to identify one or more gestures performed by the user that include one or more gestures that include movement of the user's head. In still other embodiments, the system comprises one or more gyroscopes and/or one or more accelerometers, disposed on any other portion of the user's body, configured to identify any gesture performed by the user using the other portion of the user's body (e.g., arm, hand, leg, foot, etc.). In various embodiments, the system comprises any other suitable sensor for identifying one or more gestures performed by the user.

In particular embodiments, one or more of the sensors may be detachable from the eyewear. For instance, if a wearer does not need a temperature sensor or other particular sensor, the sensor may be removed from the eyewear. In still other embodiments, some of the one or more sensors may be coupled to the handheld wireless device, while other of the one or more sensors may be coupled to the wearable device.

### More Detailed Description of System Functionality

Various embodiments of systems for monitoring the behavior patterns of an individual, the monitoring the gait of an individual and for monitoring an individual's compliance with a weight loss program are described below and may be implemented in any suitable context.

### Behavior Pattern Analysis Functionality

As noted above, a system, according to various embodiments, is adapted to monitor one or more patterns of behavior and/or one or more locations of a user of a wearable device. Various aspects of the system's functionality may be executed by certain system modules, including the behavior pattern analysis module 400A. The behavior pattern analysis module 400A is discussed in greater detail below.

Figure 4A is a flow chart of operations performed by an exemplary Behavior Pattern Analysis Module 400A, which may, for example, run on the pattern analysis server 120A, or any suitable computing device (such as the one or more health monitoring devices 156A, a handheld computing device coupled to communicate with the one or more health monitoring devices 156A or a suitable mobile computing device). In particular embodiments, the behavior pattern analysis module 400A may assess a user's behavior and determine the user's location to provide this information to the user or to a third party.

The system begins, in various embodiments, at Step 405A by providing a user with computerized eyewear comprising at least one sensor for monitoring one or more behaviors of the user and/or any suitable attributes of the user. In various embodiments, the at least one sensor may include a location sensor (e.g., a GPS unit), an accelerometer, a heart rate monitor, one or more electrocardiogram (EKG), an electroencephalogram (EEG), a pedometer, a thermometer, a front-facing camera, an eye-facing camera, a microphone, an accelerometer, a blood pressure sensor, a pulse oximeter, a near-field communication sensor, a motion sensor, a gyroscope, a geomagnetic sensor, an impact sensor, and/or any other suitable sensor. In particular embodiments, the computerized eyewear further comprises: a motion sensor, an accelerometer, a GPS unit, a gyroscope, and/or a front-facing camera.

In particular embodiments, the sensors may be coupled to the eyewear in any suitable way. For example, in various embodiments, the sensors may be physically embedded into, or otherwise coupled to the eyewear. In some embodiments, the sensors may be positioned: (1) along the brow bar of the eyewear; (2) along the temples of the eyewear; (3) adjacent the lenses of the eyewear; and/or (4) in any other suitable location.

In particular embodiments: (1) the sensors are coupled to a wireless (e.g., Bluetooth, near-field communications, Wi-Fi, etc.) device that is configured to transmit one or more signals obtained from the one or more sensors to a handheld wireless device (e.g., a smartphone, a tablet, an automated dialing device, etc.); and (2) the step of receiving one or more signals from the one or more sensors further comprises receiving the one or more signals from the wireless handheld device via the Internet. In particular embodiments, one or more of the sensors may be selectively detachable from the eyewear, or other wearable device. For example, if a user does not need the temperature sensor, the temperature sensor may be selectively removed from the eyewear and stored for future use.

At Step 410A, the system receives data generated by the at least one sensor. In particular embodiments, the data generated by the at least one sensor may include data for a heart rate, a heart rhythm or electrical activity, a brain wave activity, a distance traveled, a temperature, an image, a sound, a speed traveled, a blood pressure, an oxygen saturation level, a near-field communication, a motion, an orientation, a geomagnetic field, a global position, an impact, a medicine regime, or any other suitable data.

In various embodiments, the system may receive the data substantially automatically after the sensor generates the data. In some embodiments, the system may receive the data periodically (e.g., by the second, by the minute, hourly, daily, etc.). For example, the system may receive the data every thirty seconds throughout the day. In other embodiments, the system may receive the data after receiving an indication from the user or a third party that the system should receive the data. For instance, the user may speak a voice command to the wearable device requesting that the device track the user's steps taken. In various embodiments, the system may receive an indication from the user or a third party of when to have the system receive the data. For example, the system may receive an indication from the third party to have the system receive global positioning data at 8:00 a.m. and at 2:00 p.m.

In particular embodiments, the system may receive an indication from the user or a third party to have particular data received from a particular sensor at the same time that the system receives second particular data from a second particular sensor. For example, when the system receives data that indicates that the user's speed has increased, the system may at least partially in response to receiving the increased speed data, also obtain global position data of the user. In particular embodiments, the system may receive behavior data during a predefined time period. For instance, the system may receive behavior data for the user during a predefined time period when the user should not be moving (e.g., 11:00 p.m. through 7:00 a.m. because the user should be sleeping). In various embodiments, the system may receive the data when a sensor detects movement of the user. For example, the system may receive data from the global positioning system sensor when the accelerometer or the gyroscope detects movement of the user.

In other embodiments, the data generated by the at least one sensor may be whether the user experiences one of sudden acceleration and sudden impact. In still other embodiments, the data generated by the at least one sensor may be a heartbeat and whether the user is breathing. In yet other embodiments, the data generated by the at least one sensor may be a medicine regime associated with the user. For instance, the user or the user's physician may manually input a medicine regime into the system by stating the name of the medicine while the user or the user's physician requests that the front-facing camera capture an image of the medicine and the medicine bottle. In some embodiments, the received data generated by the at least one sensor may be one or more images captured by the forward facing camera. In other embodiments, the received data generated by the at least one sensor may be the level of one or more medicines in the user's bloodstream.

In various embodiments, the system may receive data from a single sensor. In other embodiments, the system may receive data from all of the sensors. In yet other embodiments, the system may receive multiple data from each of the sensors. In various embodiments, the system may be configured to receive first data from a first sensor at the same time that it receives second data from a second sensor. For example, the system may be configured to receive a global position from the global positioning system sensor at the same time that it receives impact data from the impact sensor.

In particular embodiments, the system may store the received data. In various embodiments, the system may store the received data substantially automatically after receiving the data. In other embodiments, the system may store the received data after receiving manual input from the user or a third party requesting that the system store the data. In various embodiments, the system may store the received data for a specified period of time. For instance, the system may store the received data for a day, a month, a year, etc., in the behavior information database 140A. In some embodiments, the system may store the received data on any suitable server, database, or device. In other embodiments, the system may store the received data on the pattern analysis server 120A. In still other embodiments, the system may store the received data in an account associated with the user. In various embodiments, the system may store the received data with a timestamp of when the data was received.

At Step 415A, the system analyzes the data generated by the at least one sensor. In various embodiments, the system analyzes the data generated by the at least one sensor substantially automatically after receiving the generated data. In various embodiments, the system may analyze the data periodically (e.g., by the second, by the minute, hourly, daily, etc.). For example, the system may analyze the data every thirty seconds throughout the day. In other embodiments, the system may analyze the data after receiving an indication from the user or a third party that the system should analyze data. For instance, the user may speak a voice command to the wearable device requesting that the device analyze the user's steps taken. In various embodiments, the system may receive an indication from the user or a third party of when to have the system analyze the data. For example, the system may receive an indication from the third party to have the system analyze global positioning data at 8:00 a.m. and at 2:00 p.m.

In other embodiments, the system may analyze the data to determine one or more of (1) the type of medicine taken by the user; (2) the time the medicine is taken by the user; and (3) the dose of medicine taken by the user. In still other embodiments, the step of analyzing the received data further comprises detecting one or more pills in the one or more images, comparing the one or more detected pills found in the one or more images to known images of pills stored in a database, identifying the one or more pills by matching the one or more pills from the one or more images to the known images of pills stored in the database, and detecting the time that the image was taken. In various embodiments, the system analyzes the level of the one or more medicines in the user's bloodstream.

Then, at Step 420A, the system determines the user's current movements using the received data in order to generate one or more movement patterns for the user. In various embodiments, the system determines the user's current movements substantially automatically after receiving the data. In various embodiments, the system may determine the user's current movements periodically (e.g., by the second, by the minute, hourly, daily, etc.). For example, the system may determine the user's current movements every thirty seconds throughout the day. In other embodiments, the system may determine the user's current movements after receiving an indication from the user or a third party that the system should analyze data. For instance, the user may speak a voice command to the wearable device requesting that the device analyze the user's steps taken. In various embodiments, the system may receive an indication from the user or a third party of when to have the system analyze the data. For example, the system may receive an indication from the third party to have the system analyze global positioning data at 8:00 a.m. and at 2:00 p.m.

In various embodiments, the system determines the user's current movements by calculating the number of steps taken by the user in a particular day. In some embodiments, the system determines the user's current movements by tracking the distance traveled by the user for a particular day. In other embodiments, the system determines the user's current movements by capturing a series of images from the front-facing camera throughout the day. In still other embodiments, the system determines the user's current movements by tracking the orientation of the user using the gyroscope. In particular embodiments, the current movements of the user may include actions such as lying down, falling, wandering, sitting, standing, walking, running, convulsing, shaking, balancing, etc.

In various embodiments, the user's current movements may include the user's current location. For example, the user's current location may be an address, a geographic area, an intersection, a bus stop, a building, or any other suitable definable location. In other embodiments, the user's current movements may help to indicate the user's current status (e.g., asleep, awake, conscious, unconscious, alive, deceased, stable, good, fair, serious, critical, injured, distressed, etc.). In some embodiments, the user's current behaviors may include compliance with prescribed treatment regimes. For instance, the user's current behaviors may include that the user has not been complying with prescribed treatment regimens as captured through the front-facing camera of the user not taking prescribed medicine.

In various embodiments, the system tracks current movements, current location, current status, and current compliance to generate one or more movement patterns, location patterns, status patterns, and compliance patterns. In some embodiments, the system generates the one or more patterns substantially automatically after the system determines the user's current movements, location, status, and compliance. In some embodiments, the system may generate the patterns periodically (e.g., by the second, by the minute, hourly, daily, weekly, monthly, etc.). For example, the system may generate a movement pattern for each month. In other embodiments, the system may generate the pattern after receiving an indication from the user or a third party that the system should generate the pattern. For instance, the user may speak a voice command to the wearable device requesting that the device generate a pattern for the number of steps taken by the user for a typical day. In various embodiments, the system may receive an indication from the user or a third party of when to have the system generate the patterns. For example, the system may receive an indication from the third party to have the system generate a location pattern for the location of the user at 8:00 a.m. and at 2:00 p.m. for the previous month.

In various embodiments, the movement patterns may include one or more typical movements made by the user. For example, the movement pattern may include that the user gets out of bed every morning. In particular embodiments, the location patterns may include one or more typical locations of the user. For instance, the location pattern may include that the user is at a first particular address in the morning, at a second particular address during the day, and at the first particular address at night. In some embodiments, the status patterns may include one or more typical statuses of the user. In example, the status pattern may include that the user is awake from 7:00 a.m. until 11:00 p.m. and asleep from 11:00 p.m. until 7:00 a.m. In other embodiments, the compliance patterns may include one or more typical compliance schedules of the user. For example, the compliance pattern may include that the user is prescribed a medicine that the user takes every day in the morning with food. In yet other embodiments, the medicine regime patterns may include one or more typical medicine regimes for the user. For instance, the medicine regime pattern may include that the user takes a particular yellow pill, a particular white pill, and a particular pink pill in the evening with food. In various embodiments, the system may include one or more typical levels of one or more medicines in the user's bloodstream. For example, the typical level of a particular medicine in the user's bloodstream may be a certain volume at a particular period of time.

In particular embodiments, the system may store the generated patterns in an account associated with the user. In some embodiments, the generated patterns may be accessible by the user or a third party. For instance, the generated patterns may be diagramed in a chart that is accessible from the wearable device or from a computing device by the user's physician. In various embodiments, the system may store the generated patterns in the behavior information database 140A. In particular embodiments, the system may store information in the behavior information database 140A regarding past movement patterns associated with the user (e.g., when the user goes into different rooms in the user's house, when the user eats, when the user takes a walk, the destinations along the walk, etc.). In some embodiments, the system may store information in the behavior information database 140A regarding the user's sleep patterns. In other embodiments, the system may store information in the behavior information database 140A regarding geo-fences associated with the user. In still other embodiments, the system may store information in the behavior information database 140A regarding deviations to the user's typical behavior (e.g., movement) patterns.

At Step 425A, the system compares the user's behaviors (e.g., movements) to the previously established one or more patterns for the user. In some embodiments, the system compares the user's movement to the previously established one or more movement patterns for the user substantially automatically after the system receives the user's current movements. In some embodiments, the system may compare the user's movement to the previously established one or more movement patterns periodically (e.g., by the second, by the minute, hourly, daily, weekly, monthly, etc.). For example, the system may compare the user's current movement to the previously established one or more movement patterns every thirty minutes throughout the day. In other embodiments, the system may compare the user's movement to the previously established one or more movement patterns after receiving an indication from the user or a third party that the system should compare the user's movement to the previously established movement pattern. For instance, the user may speak a voice command to the wearable device requesting that the device compare the user's movements for the current day to a movement pattern established the previous month. In various embodiments, the system may receive an indication from the user or a third party of when to have the system compare the user's movements to the one or more patterns. For example, the system may receive an indication from the third party to have the system compare the user's current location to a location pattern for the location of the user at 8:00 a.m. and at 2:00 p.m. on a typical day.

In some embodiments, the system may compare the user's movements to a previously established movement pattern by calculating the number of steps taken by the user in the particular day to a predetermined average number of steps taken by the user in a day. In various embodiments, the system may compare the user's location to a previously established location pattern by determining the average location of the user at a particular time of day. In other embodiments, the system may compare the user's status to a previously established status pattern by determining the user's average status at particular times of day.

In still other embodiments, the system may compare the user's compliance with a prescribed treatment regime by determining the user's average compliance with the prescribed treatment regime for a particular day. In yet other embodiments, the system may compare the one or more of the type of medicine taken; the time the medicine is taken; and the dose of the medicine taken to the stored medicine regime for the user. In various embodiments, the system may compare the level of one or more medicines in the user's bloodstream by determining the average level of the one or more medicines in the user's bloodstream at particular times of day.

In particular embodiments, the system may store the comparisons in an account associated with the user. In some embodiments, the comparisons may be accessible by the user or a third party. For instance, the comparisons may be diagramed in a chart that is accessible from the wearable device or from a computing device by the user's physician.

Continuing to Step 430A, the system detects one or more inconsistencies between the user's current movements as compared to the previously established one or more patterns. In other embodiments, the system does not detect one or more inconsistencies between the user's current movements as compared to the previously established one or more patterns. In various embodiments, the system may detect the one or more inconsistencies by determining that the user's current movements are inconsistent with the previously established patterns. In particular embodiments, the user's current movements may be inconsistent with previously established patterns based on the current movements being different from the established patterns by a particular percentage. For instance, where the user's movement patterns establish that the user walks a total of one mile a day, the system may determine that the user's current movement of walking ½ mile for the day is inconsistent with the user's previously established pattern of walking one mile a day because there is a difference of 50%.

In some embodiments, the user's current movements may be inconsistent with the previously established movement patterns based on the user's current movements not matching the previously established movement patterns. For instance, for the movement pattern that includes that the user gets out of bed every morning, where the system detects that the user does not get out of bed on a particular morning, the system may determine that the user's current movements are inconsistent with the previously established pattern.

In other embodiments, the user's current movements may be inconsistent with the previously established patterns based on the user's current location not matching the previously established location patterns. For example, for the location pattern that includes the user at a first particular address in the morning, at a second particular address during the day, and at the first particular address at night, where the system detects that the user was not at the second particular address during the day, the system may determine that the user's current movements are inconsistent with the previously established pattern.

In still other embodiments, the user's current movements may be inconsistent with the previously established patterns based on the user's current status not matching the previously established status patterns. For instance, for the status pattern that includes that the user is awake from 7:00 a.m. until 11:00 p.m. and asleep from 11:00 p.m. until 7:00 a.m., where the system detects that the user is asleep from 7:00 a.m. until 2:00 p.m., the system may determine that the user's current movements are inconsistent with the previously established pattern.

In yet other embodiments, the system may detect one or more inconsistencies between the medicine regime associated with the user and the determined one or more of the type of medicine taken by the user, the time the medicine is taken by the user, and the dose of medicine taken by the user. For instance, for a medicine regime that includes that the user takes a particular pill having a particular color (e.g., yellow), shape (e.g., triangular, square), and marking (e.g., the number 17) in the evening with food, where the system detects that the user did not take the particular yellow pill on a particular evening with food, the system may determine that the user's current movements are inconsistent with the previously established pattern.

In some embodiments, the system may detect one or more inconsistencies between the level of the one or more medicines in the user's bloodstream and the determined typical level of the one or more medicines in the user's bloodstream. For example, for a typical level of a particular medicine in the user's bloodstream that includes that the level is a certain volume at a particular period of time, where the system detects that the level of the medicine in the user's bloodstream is less than the typical level, the system may determine that the user's current movements are inconsistent with the previously established patterns.

At Step 435A, the system notifies the user and/or a third party of the detected one or more inconsistencies. In particular embodiments, in addition to notifying at least one recipient selected from a group consisting of: the user and the third party, the system updates the user's account to note that a notification was sent. In various embodiments, the system notifies the user of the detected one or more inconsistencies. In some embodiments, the system notifies the third party of the detected one or more inconsistencies. In particular embodiments, the system may notify the user of the detected one or more inconsistencies by displaying an image on the lens of the eyewear, or on another display associated with the eyewear. In other embodiments, the system notifies the user of the one or more inconsistencies by communicating through a speaker to the user.

In various embodiments, the third party may be a relative of the user. In other embodiments, the third party may be a police department. In particular embodiments, the third party may be an ambulance service. In some embodiments, the third party may be a physician. In still other embodiments, the third party may be an independent living provider. In yet other embodiments, the third party may be a particular caregiver of the user.

In some embodiments, the system notifies the user and/or the third party of the one or more inconsistencies by sending a notification to the user's and/or the third party's mobile devices. In particular embodiments, the system notifies the user and/or the third party of the one or more inconsistencies by email or text message. In other embodiments, the system may notify the user and/or the third party of a single inconsistency substantially immediately after the system detects the inconsistency between the user's current movements as compared to the previously established one or more movement patterns. In yet other embodiments, the system may notify the user and/or the third party of all inconsistencies detected on a particular day at the end of that day.

In various embodiments, the system may notify the user and/or the third party of the one or more inconsistencies after a particular event. For example, the system may notify the user if the system determines that the calculated number of steps of the user for a particular day is less than a predetermined percentage of the predetermined average number of steps taken by the user in a day. In some embodiments, the system may notify the user and/or the third party of the one or more inconsistencies after a particular period of time. For instance, the system may notify the third party of an association one hour after the system detects one or more inconsistencies between the user's current movements as compared to the previously established one or more movement patterns. In still other embodiments, the system may notify the user of the one or more inconsistencies at a particular time of day. As an example, the system may notify the user of one or more inconsistencies between the user's current movements as compared to the previously established one or more movement patterns at the end of the day.

In various embodiments, at least partially in response to detecting whether the user moves during the predefined time period, the system may notify the user and/or third party if the user does not move during the predefined time period. In other embodiments, at least partially in response to detecting one of sudden acceleration and sudden impact (e.g., such as that associated with a fall), the system may notify user and/or the third party that the user experienced the one of sudden acceleration and sudden impact. In some embodiments, at least partially in response to not detecting one of a heartbeat or breathing associated with the user, the system may notify the user and/or the third party that the heartbeat and/or breathing of the user cannot be detected. This may indicate, for example, a medical emergency associated with the user or a malfunction of one or more system components.

In particular embodiments, the system may notify the user and/or the third party of detected inconsistencies between the user's current movements and the previously established movement patterns. In some embodiments, the system may notify the user and/or the third party of detected inconsistencies between the user's current location and the previously established location patterns. In other embodiments, the system may notify the user and/or the third party of detected inconsistencies between the user's current status and the previously established status patterns. In still other embodiments, the system may notify the user and/or the third party of detected inconsistencies between the user's current compliance and the previously established compliance patterns. In yet other embodiments, the system may notify the user and/or the third party of detected inconsistencies between the user's current medicine regime and the previously established medicine regime patterns. In various embodiments, the system may notify at least one recipient selected from a group consisting of: the user and the third party of detected inconsistencies between the user's current level of one or more medicines and the previously established typical one or more levels of medicine.

In particular embodiments, the system may notify the user and/or the third party of detected inconsistencies between the stored medicine regime and the one or more of the type of medicine taken, the time the medicine is taken, and the dose of medicine taken. In some embodiments, the system may notify at least one recipient selected from a group consisting of: the user and the third party if the user removes the wearable device for a predetermined period of time. In other embodiments, the system may notify the user and/or the third party if the user does not consume food for a predetermined period of time. In particular embodiments, the system may notify the user and/or the third party if the user does not consume liquids for a predetermined period of time. In various embodiments, the system may notify the user and/or the third party if the user's caloric intake is above or below a predetermined number of calories. In some embodiments, the system may notify the user and/or the third party if the user's oxygen levels fall below a predetermined threshold. In other embodiments, the system may notify the user and/or the third party if the user's blood sugar drops below a predetermined threshold.

In various embodiments, the system, when executing the Behavior Pattern Analysis Module 300, may omit particular steps, perform particular steps in an order other than the order presented above, or perform additional steps not discussed directly above.

### Gait Monitoring Functionality

Various embodiments of a system for the monitoring the gait of an individual are described below and may be implemented in any suitable context. For example, particular embodiments may be implemented to: (1) identify one or more medical conditions associated with the individual; (2) assess the fit of a prosthetic device worn by the individual, and/or (3) assess an individual's recovery from a particular injury or medical procedure.

Various aspects of the system's functionality may be executed by certain system modules, including the gait monitoring module 400B. The gait monitoring module 400B is discussed in greater detail below.

Referring to Figure 4B, when executing the gait monitoring module 400B, the system begins, in various embodiments, at Step 405B by receiving data from a wearable device worn by an individual whose gait is to be monitored by the system. In particular embodiments, the system is configured to receive data from one or more sensors (e.g. an accelerometer, a gyroscope, a position locating device and/or magnetometer) while: (1) the individual is wearing the wearable device adjacent the user's face and/or head; and (2) the individual is walking or running. In particular embodiments, the system is configured to receive the data while the individual is walking or running within the context of their typical daily routine, and not within the context of a medical diagnostic visit. The system may also or alternatively be configured to receive data within the context of a medical diagnostic visit (e.g., at a doctor's office, hospital, or other medical facility).

In particular embodiments, the one or more of the system's sensors may be embedded in, or otherwise attached to, eyewear or other wearable device (e.g., another wearable device worn adjacent the individual's head or another suitable part of the individual's body). In particular embodiments, at least one or more of the system's sensors may be incorporated into a prosthesis or into a portion of the individual's shoes. In certain embodiments, the system may include one or more sensors that are incorporated into (e.g., embedded in, or attached to) a plurality of wearable devices (e.g., eyewear and the individual's shoes) that are adapted to be worn simultaneously by the user while the system retrieves signals from the sensors to assess the individual's gait.

In particular embodiments, the system may include a set of eyewear that includes one or more motion sensors (e.g., accelerometers, gyroscopes, or location sensors) for sensing the movement of the head of an individual who is wearing the eyewear as the individual walks. The system may then use this head movement information (e.g., using any suitable technique, such as any suitable technique described herein) to determine whether the user has a gait abnormality. The system may do this, for example, by comparing one or more of the measured head motions of an individual (e.g., as measured when the individual is walking or running) with the actual or typical head motions experienced by individuals with gait abnormalities as those individuals walk or run.

In various embodiments, the system is configured to measure and receive at least one of the velocity, height, and orientation of one or more of the individual's feet. For example, in certain embodiments, the system is configured to measure and receive (e.g., using the suitable sensors) the linear acceleration of each of the individual's feet, the height of each of the feet from the ground, and/or the position and/or orientation of each of the feet relative to the central axis of the individual's body as the individual walks or runs.

The system continues at Step 410B by using the data received from the system's sensors to identify one or more relative peaks in linear acceleration of the individual's body and/or head as the user ambulates (e.g., walks or runs). In various embodiments, the system may do this by processing the data received from the sensor(s) in Step 405B, and then isolating the relative peaks in the data. Such peaks represent the relative maxima and minima of the linear acceleration of the user's head, body, and/or one or more of the individual's lower body parts (e.g., knee, ankle, or foot) as the user ambulates. Alternatively or additionally, the system may be configured to identify the relative peaks in linear acceleration by identifying the slope of the line formed by regression analysis of the data received from the sensors. This regression analysis may indicate the change in magnitude of the linear acceleration with time.

In identifying the relative peaks in linear acceleration, the system is further configured to identify the peaks such that the magnitude and phase of these peaks may be utilized to aid in the diagnosis of one or more gait abnormalities by comparing the magnitude and phase of the peaks associated with the individual's gait with the magnitude and phase of the peaks associated with: (1) the gait of one or more individuals who are known to have one or more gait abnormalities; (2) a typical gait associated with individuals who are known to have one or more gait abnormalities; and/or (3) the individual's normal gait (which may be determined based on data stored in system memory that the system obtained, for example, when the individual was known to walk or run without a gait abnormality). This comparison may be helpful in determining whether the individual has a gait abnormality and, if so, whether the gait abnormality exists due to an improper prosthetic fit.

In a particular embodiment, the above comparison may involve comparing the magnitude and/or phase of peaks that represent a user's head movement as the user ambulates with the magnitude and/or phase of peaks that represent the head movement, during ambulation, of (1) one or more individuals who are known to have one or more gait abnormalities; (2) a typical individual (or model theoretical individual) who is known to have one or more gait abnormalities; and/or (3) the individual themself (this data may be determined, for example, based on data stored in system memory that the system obtained, for example, when the individual was known to walk or run without a gait abnormality).

Continuing at Step 415B, the system is configured to analyze the received gait information to determine whether the individual has an identifiable gait abnormality and to communicate the results of the analysis to the user. In various embodiments, the system may use the gait information to: (1) identify potential, previously undiagnosed medical conditions (e.g., one or more medical conditions, such as ALS or MS, that may be indicated by a particular gait abnormality, such as foot drop); (2) assess the quality of the fit of a prosthesis; and/or (3) assess the individual's progress in recovering from an injury or medical procedure (e.g., knee or hip surgery).

### Use of System to Identify Previously Undiagnosed Medical Condition

In identifying a potential, previously undiagnosed medical condition, the system is configured to compare the gait of the individual with: (1) the gait of one or more individuals who are known to have one or more gait abnormalities (e.g., hemiplegic gait, diplegic gait, neuropathic gait, foot drop, myopathic gait, or ataxic gait); (2) a typical gait associated with individuals who are known to have one or more gait abnormalities; and/or (3) the individual's normal gait. To do this, the system may compare one or more gait patterns of a user (e.g., in the manner discussed above or in any other suitable way) with information regarding one or more abnormal gait patterns that is stored in a gait database 130B. The system may do this, for example, by applying any suitable mathematical or other data comparison technique to determine whether one of more of the individual's gait patterns are at least substantially similar to one or more abnormal gait patterns stored in the system's gait database 130B.

If the system determines that the individual has, or may have, a particular gait abnormality, the system may generate and send a notification to a suitable individual (e.g., the individual or the individual's physician) indicating that the individual may have a gait abnormality and/or that it may be beneficial to examine or monitor the individual for one or more medical conditions that are typically associated with the gait abnormality, e.g., stroke, amyotrophic lateral sclerosis, muscular dystrophy, Charcot Maries Tooth disease, multiple sclerosis, cerebral palsy, hereditary spastic paraplegia, and Friedrich's ataxia. The notification may be, for example, a suitable electronic notification (e.g., a message on a display screen, an e-mail, a text), or any other suitable notification..

### Use of System to Determine Whether a Prosthesis Fits Correctly

In assessing the quality of fit of the prosthesis, the system in various embodiments, may, in various embodiments, be configured to compare the user's assessed gait with: (1) the gait of one or more individuals who are known to have one or more gait abnormalities that are associated with an improper prosthetic fit; (2) a typical gait associated with individuals who are known to have one or more gait abnormalities that are associated with an improper prosthetic fit; and/or (3) the individual's normal gait. This comparison may be done as discussed above or in any other suitable way. In particular embodiments, the gait patterns that the individual's gait patterns are compared with may be modeled, for example, based on previously recorded data for individuals with one or more physical attributes (e.g., height, age, weight, femur length, etc...) that are similar to that of the individual. In various other embodiments, such patterns may be modeled from previously recorded data for users that aren't physically similar to the individual.

In response to determining that the individual has one or more gait patterns that are associated with an improper prosthetic fit, the system may generate an alert indicating that the prosthesis may fit improperly. The system may send this alert electronically, for example, via email, text message, or via a display on a display screen, to the user and/or their physician or other suitable individual.

In various embodiments, after determining that the individual has an abnormal gait, the system may then determine whether the gait deviation results from an improperly fitting prosthesis or from an injury associated with the individual (e.g., an infected wound adjacent the prosthesis) . It is noted that an improper fit of a prosthetic leg may result in any of a number of gait deviations such as trans-femoral (TF) long prosthetic step, TF excessive lumbar lordosis, TF drop off at end of stance, TF foot slap, TF medial or lateral whips, TF uneven heel rise, etc. While such gait deviations may result from an improper prosthetic fit, they may also manifest from: (1) various improper actions or movements by the amputee while the amputee is wearing the prosthesis; or (2) an injury adjacent the prosthesis. Clinically distinguishing an improper gait caused by a poorly fitting prosthetic from an improper gait caused by improper use of a properly fitted prosthesis may be important in helping the amputee regain proper functionality of the prosthetic.

### Use of System to Assess an Individual's Recovery from an Injury or Medical Procedure

In assessing an individual's recovery from an injury or medical procedure, the system may compare the individual's current gait with historical gait information for the individual stored in the database 130B. The historical gait information, in various embodiments, may include gait pattern information taken for the individual at some time in the past (e.g., the recent past) before or after the user suffered the injury or underwent the medical procedure.

The system may then analyze both sets of gait information to determine whether the individual's gait has become more consistent with the user's normal gait (e.g., fewer abnormalities in gait, more regular, quicker lateral acceleration, etc.) To do this, the system may, in various embodiments, compare the user's current gait information with a normal gait to determine whether the user's gait has become more consistent with a normal gait over time. In other embodiments, the system may compare the most current gait data with other post-procedure or post-injury gait data for the individual to determine whether the user's gait has become more consistent with a normal gait (e.g., the individual's normal gait).

Upon analyzing both sets of gait information, the system may generate an appropriate assessment of the user's recovery and/or to generate one or more treatment recommendations. The system may, in various embodiments, generate a report that communicates the progress of an individual's recovery. The system may also, or alternatively, generate an alternate treatment plan for the individual, if necessary. For example, a particular generated report may include one or more recommendations with regard to a particular type and length of physical therapy to be performed by the individual, and/or one or more dietary restrictions that the individual should implement to aid recovery to regain muscle tone and strength in the affected limb. The system may then communicate the report to the individual or an appropriate third party.

### Weight Loss Compliance Module

Figure 4C is a flow chart of operations performed by an exemplary weight loss compliance module 400C, which may, for example, run on the compliance server 120C, or any suitable computing device (such as the one or more health monitoring devices 156C, a handheld computing device coupled to communicate with the one or more health monitoring devices 156C or a suitable mobile computing device). In particular embodiments, the weight loss compliance module 400C may assess a wearer's compliance with a weight loss plan (e.g., a dietary plan, an exercise plan, a sleep plan, and/or a medicine regime) and provide recommendations, feedback and/or supportive messages on how the wearer can improve their compliance with the weight loss plan.

The system begins, in various embodiments, at Step 405C where the system receives, by at least one processor, at least one signal generated by one or more sensors operatively coupled to the computerized eyewear. In various embodiments, the at least one signal received may include one or more images taken by a forward facing camera coupled to the health monitoring device 156C. In other embodiments, the at least one signal received may include one or more signals from a global positioning system unit that are associated with the location of the wearer of the health monitoring device 156C. In still other embodiments, the at least one signal received may include one or more signals from an olfactory sensor that may be used to determine the smell associated with one or more foods being prepared for ingestion by the wearer of the health monitoring device 156C. In yet other embodiments, the at least one signal received may include one or more audio signals received by a microphone coupled to the health monitoring device 156C.

In various embodiments, the system receives the signals substantially automatically after the sensor generates the signal. In some embodiments, the system may receive the signal periodically (e.g., by the second, by the minute, hourly, daily, etc.). For example, the system may receive the signal every thirty seconds throughout the day. In other embodiments, the system may receive the signal after receiving an indication from the wearer or a third party that the system should receive the signal. For instance, the wearer may speak a voice command to the wearable device requesting that the device track the food being prepared by the wearer. In various embodiments, the system may receive an indication from the wearer or a third party of when to have the system receive the signal. In particular embodiments, the system may receive an indication from the wearer or a third party to have particular data received from a particular sensor at the same time that the system receives second particular data from a second particular sensor. For example, when the system receives one or more images, the system may at least partially in response to receiving the one or more images, also obtain global position data of the wearer, olfactory data associated with food being prepared by the wearer, etc.

In particular embodiments, the system may store data associated with the received signal in memory (e.g., local memory, remote memory, etc.). In various embodiments, the system may store the data substantially automatically after receiving the signal. In other embodiments, the system may store the data after receiving manual input from the wearer or a third party requesting that the system store the data. In various embodiments, the system may store the data for a specified period of time. For instance, the system may store the data for a day, a month, a year, etc., in, for example, the dietary information database 140C, the exercise information database 142C, the medicine database 144C, and/or the sleep information database 146C. In some embodiments, the system may store the data on any suitable server, database, or device. In other embodiments, the system may store the data on the compliance server 120C. In still other embodiments, the system may store data in an account associated with the wearer. In various embodiments, the system may store the data with a timestamp of when the data was received.

At Step 410C, the system, at least partially in response to receiving the at least one signal from the one or more sensors, determines, by at least one processor, an identity of a food that the wearer is preparing to ingest. In various embodiments, the at least one signal may be an image that is captured by a forward facing camera. In other embodiments, the at least one signal may be a signal received from a GPS chip that provides the current location of the wearer. In still other embodiments, the at least one signal may be an audio signal received from a microphone. In yet other embodiments, the signal may be a smell captured by the olfactory sensor.

In embodiments, where the at least one signal is one or more captured images, the system may capture an image of packaging associated with a food that is being prepared by the wearer. In some such embodiments, the system may analyze the image to detect a barcode located on the packaging associated with the food. Once the system detects the barcode, the system may decipher the bar code and search the dietary information database 140C of barcodes to find a matching barcode. Once the system identifies the food associated with the barcode, the system may also obtain from the dietary information database 140C nutritional information (e.g., calories, fat content, protein content, etc.) that is associated with the matching barcode.

In other embodiments, the system may analyze the captured one or more images to identify a food located in the captured one or more images. For example, the system may capture an image of a hamburger that the wearer is preparing to eat. The system may search the dietary information database 140C of images of food to find a matching image of a hamburger that is similar (e.g., the same, substantially similar, etc.) to the hamburger that the wearer is preparing. Once a match is determined by the system, the system can identify the food and obtain the nutritional information associated with matching image stored in the dietary information database 140C. In various embodiments, the system may capture a series of images that show the wearer preparing the hamburger. Thus, in some embodiments, the system may first capture an image of the hamburger as the wearer barbecues the hamburger. From the first image, the system may identify the hamburger by finding a matching image of a hamburger in the dietary information database 140C. Next, the system may capture one or more images of the user removing a hamburger roll from a package of hamburger rolls. The system may detect a barcode from the image of the packaging of the hamburger rolls and identify the roll and the nutritional information associated with the roll from the barcode associated with the one or more images by searching the dietary information database 140C for a matching barcode. The system may continue to identify various other condiments that the wearer places on the hamburger in a similar fashion.

In still other embodiments, the system may capture an image of a food that the wearer is preparing to ingest while the system simultaneously receives a GPS signal from a GPS chip contained in the computerized eyewear worn by the wearer. In some such embodiments, the system may determine the location of the wearer from the GPS signal, determine if a restaurant or similar food establishment is located at the location, and simultaneously analyze one or more captured images to identify the food that the wearer is preparing to ingest by, for example, one of the methods described above. In some of these embodiments, the wearer's location and a determination of a particular restaurant or similar food establishment may assist the system in identifying the food that the wearer is preparing to ingest. For example, if the system cannot positively identify the exact food item the wearer is preparing to ingest, the system may display a list of one or more food items associated with the restaurant or similar food establishment on a display associated with the computerized eyewear (e.g., a display coupled to the computerized eyewear, a display on a handheld computing device, etc.) and allow the wearer to select the particular food item from the displayed list of one or more food items.

In still other embodiments, the system may receive an audio signal from one or more microphones coupled to the computerized eyewear. In some embodiments, the system may convert the received audio signal to text and use the text to search the dietary information database 140C for matching text words to identify the food that the wearer is preparing to ingest. In some embodiments, the dietary information database 140C may also contain nutritional information associated with each word in the database. For example, the wearer may place the computerized eyewear in a state where it is ready to accept voice input. The wearer may, for example, say "hamburger". The system may convert the received voice command to text and search the dietary information database 140C for a matching word. Each word in the database may contain associated nutritional information. Thus, if the system finds a match for the word "hamburger", the system identifies the food item as a "hamburger" and obtains nutritional information associated with the word "hamburger." In some embodiments, once the system converts the received audio signal into text, the system may seek confirmation of the converted word by the wearer before searching the database of food items.

As noted above, in various embodiments, the system may determine a wearer's location using a signal received from the GPS chip in the computerized eyewear or from a GPS chip on a handheld computing device coupled to the computerized eyewear. In some such embodiments, if the system detects that the user is located in a restaurant or other food establishment, the system may begin to monitor the wearer's speech in order to detect one or more food items ordered by the wearer. As the system detects the wearer's speech, the system may convert the speech to text and search the dietary information database 140 based on one or more of: (1) the location of the wearer, (2) the associated name of the restaurant or other similar food establishment, or (3) a detected name of a food item ordered by the wearer. In various embodiments, the system may automatically detect the ordered food item. In other embodiments, once the system identifies an ordered item, the system may request confirmation that the detected item is accurate prior to making a final identification of the food item that the wearer is preparing to ingest.

In various embodiments, confirmation of the identity of the food item may be made by the wearer orally using the microphone on the computerized eyewear. In other embodiments, the system may request confirmation by displaying the identified food item on a display associated with the computerized eyewear. For example, the computerized eyewear may contain a built in display that the wearer can view while wearing the computerized eyewear. In other embodiments, the computerized eyewear may be coupled (e.g., wirelessly, wired, etc.) to a handheld computing device that displays the selected food item. In some such embodiments, the system may be configured to allow the user to select a "confirm" or "deny" button displayed on the handheld computing device.

At Step 415C, the system determines, by at least one processor, a quantity of the food that the wearer is preparing to ingest. In various embodiments where the system detects and reads a barcode located in one or more captured images, the system may determine the serving size associated with the barcode by searching the dietary information database 140C for a matching barcode and nutritional information, which may also include a serving size for the food item. For example, in some instances where the wearer is eating prepackaged food, the matching barcode may indicate that the serving size associated with the packaged food is a single serving (e.g., a candy bar, a frozen meal, etc.). In other embodiments, the matching barcode may indicate that the serving size is a teaspoon, a tablespoon, a cup, etc.

In various embodiments where the serving size is not a single serving, the system may calculate an estimated volume from the captured one or more image. In other embodiments, the system may capture and analyze one or more images of the food and calculate an estimate of the quantity of the food that the wearer is preparing to ingest. For example, in some embodiments, the system may analyze the captured one or more images to count the number of teaspoons, tablespoons or cups of a food that the wearer selects. In other embodiments, the wearer may input the quantity of food using an input device (e.g, a food scale) coupled to the computerized eyewear or a handheld computing device coupled to the computerized eyewear or the wearer may input the quantity using an audio input via the one or more microphones coupled to the computerized eyewear.

In other embodiments, the system may analyze the captured one or more images to detect a vessel (e.g., bowl, measuring cup, container, etc.) that the wearer is using to measure a quantity of food. In some embodiments, the system may determine the quantity of the selected food based on the identity of the vessel. For example, a set of measuring cups and/or spoons may be color coded based on the size of each cup or spoon. In these embodiments, the system may analyze the captured one or more images to detect the particular measuring cup and/or spoon being used. In other embodiments, the system may analyze the captured one or more images to detect one or more markings located on the vessel that would allow the system to determine an accurate measurement of the food based on the identity of the vessel and/or the one or more markings on the vessel. In yet other embodiments, the system may be configured to operatively couple (e.g. via Bluetooth, Wi-Fi, physical wiring, etc.) to a measuring device such as a scale, an electronic measuring cup, etc., and use a received signal from the measuring device to determine the quantity of the selected food.

At Step 420C, the system tracks, by at least one processor, the identity and the quantity of the food that the wearer is preparing to ingest. In various embodiments, the system may store the identity and quantity of a food that a wearer prepares to ingest in an account associated with the wearer on the system. In some embodiments, the system may allow the user to input when the wearer plans on eating the prepared food (e.g., for breakfast, lunch, dinner, midmorning snack, late afternoon snack, etc.). In other embodiments, the system may determine a date and time that the wearer ingests the prepared food. For example, the system may determine the date, time, and associated meal (e.g., breakfast, lunch, dinner, etc.) and stored the information in a database (e.g., dietary information database 140C) or an account on the system associated with the wearer. In still other embodiments, the system may track the number of calories associated with the prepared food, the nutritional information (e.g., amount of fat, protein, carbohydrates, vitamins, minerals, etc.) associated with the prepared food, and also store this information in the database or account associated with the wearer.

At step 425C, the system compares, by at least one processor, the identity and quantity of the food that the wearer is preparing to ingest to a predetermined weight loss plan. In various embodiments, the weight loss plan may contain one or more of a dietary plan, an exercise plan, a medicine regime, or a sleep plan. In some embodiments, the weight loss plan may be prepared by the wearer. In other embodiments, the weight loss plan may be prepared by a third-party (e.g., a doctor, a trainer, a nutritionist, etc.).

In various embodiments, the dietary plan for the wearer may consist of at least one of: (A) one or more daily values for: (1) total calorie intake, (2) total fat intake, (3) total protein intake, (4) total carbohydrate intake; (B) one or more prohibited foods; (C) one or more prohibited vitamins; (D) one or more prohibited minerals; (E) a daily expected weight loss value; (F) a weekly expected weight loss value; (G) a recommended amount of daily sleep; (H) etc. In various embodiments, the system may compare the amount and quantity of food and its associated nutritional information to the dietary plan values in order to determine if the wearer is complying with the dietary plan. In some embodiments, the system may compare the actual consumed total daily calorie intake to the planned total daily calorie intake. In other embodiments, the system may, in addition to the total daily calorie intake also compare one or more of the actual daily protein intake, daily fat intake or daily carbohydrate intake to the planned daily values.

In various embodiments, the exercise plan may consist of at least one of: (A) a weight loss goal; (B) a target daily calories to burn in order to lose a particular amount of weight per week; (C) various suggested exercises to achieve the desired weight loss goal; or (D) one or more recommended daily physical activities (e.g., walking, stretching, etc.). In some such embodiments, the system may be configured to track one or more of the wearer's (1) movements, (2) heart rate, or (3) respiration rate to determine the amount of calories burned by the wearer and the type of exercise performed by the wearer.

In various embodiments, the system may use signals received from the one or more sensors (e.g., gyroscope, accelerometer, GPS unit, heart rate sensor, etc.) to determine the wearer's movements and calories burned. In some embodiments, the system may determine the wearer's current movements by tracking the distance traveled by the wearer for a particular day. In still other embodiments, the system may determine the wearer's current movements by tracking the orientation of the wearer using the gyroscope. In particular embodiments, the current movements of the wearer may include actions such as running, jumping, bicycling, weight lifting, sitting, standing, etc. In various embodiments, the system may be configured to determine a total calories burned by the wearer for any particular activity based on one or more of (1) the movements associated with the wearer during the activity, (2) the amount of elapsed time during which the activity is performed, (3) the wearer's heart rate (actual, average, mean, etc.) during the time the activity is performed and/or after completion of the activity, or (4) the wearer's respiration rate during and/or after the activity is performed by the wearer.

In various embodiments, the system determines the wearer's current movements, heart rate, and/or respiration rate substantially automatically after receiving the signals from the one or more sensors. In various embodiments, the system may determine the wearer's current movements, heart rate, and/or respiration rate periodically (e.g., by the second, by the minute, hourly, daily, etc.). For example, the system may determine the wearer's current movements, heart rate, and/or respiration rate every thirty seconds throughout the day. In other embodiments, the system may determine the wearer's current movements, heart rate, and/or respiration rate after receiving an indication from the wearer that the system should analyze the signals received from the one or more sensors. For instance, the wearer may speak a voice command to the computerized eyewear requesting that the device analyze the wearer's steps taken, heart rate and/or respiration rate. In other embodiments, the wearer may indicate to the system using a handheld computing device operatively coupled to the computerized eyewear that the system should capture the wearer's current movements, heart rate, and/or respiration rate.

In various embodiments, the weight loss plan may contain a medicine regime that is designed to support the wearer's weight loss goals. For example, the medicine regime may comprise at least one of (A) one or more prescription weight loss medicines; (B) one or more over the counter weight loss medicines; or (C) one or more over the counter supplements (e.g. Chitosan, Chromium Picolinate, etc.). In various embodiments, the medicine regime may also comprise tracking one or more prescription medicines that the wearer is already taking to determine any weight loss or weight gain side effects that may be caused by the one or prescribed medications taken by the wearer. For example, if the wearer is diabetic, the medicine regime may track the wearer's use of insulin in conjunction with the wearer's food intake, physical activity, and glucose levels to determine any weight loss or weight gain effects on the wearer.

In some embodiments, the wearer or a third-party (e.g., the wearer's physician, etc.) may manually input a medicine regime into an account associated with the wearer on the system. For example, where a physician prescribes one or more weight loss medicines or supplements as part of the wearer's weight loss plan, the prescribed medicines or supplements may be entered into the wearer's weight loss plan. In other embodiments where the wearer is already taking prescription medications, the wearer may manually enter one or more prescription medicines into the wearer's account on the system or the wearer's account may be linked to a medical records database so that prescribed medicines are automatically linked to the wearer's weight loss plan.

In still other embodiments, the one or more sensors on the computerized eyewear may capture one or more images of the one or more medicines/supplements that the wearer is preparing to ingest. The system may be configured to analyze the captured image to detect the presence of a label on a medicine/supplement bottle to identify the medicine/supplement and dose being ingested by the wearer. In some embodiments the system may perform optical character recognition techniques on the label to identify the type of medicine/supplement and the dose taken by the wearer. Once the medicine/supplement and/or dose is identified, the system may add the medicine/supplement to the wearer's medicine regime and perform a look-up of the medicine/supplement in a medicine database 144C to determine if the medicine/supplement has any positive or negative side effect on weight loss. Once the system receives or identifies all medicines/supplements taken by the wearer, the system may track the wearer's compliance with the medicine regime.

In various embodiments, the system may capture one or more images throughout the day and analyze the images to determine one or more of (1) a type of medicine/supplement taken by the wearer; (2) the time the medicine/supplement is ingested by the wearer; and (3) the dose of medicine/supplement ingested by the wearer. For example, in some embodiments, the system may detect one or more pills in the captured one or more images, compare the one or more detected pills found in the captured one or more images to known images of pills stored in the medicine database 144C, identify the one or more pills by matching the one or more pills from the captured one or more images to the known images of pills stored in the medicine database 144C, and detect the time that the captured one or more images was taken. Based on this information, the system is able to determine if the wearer is complying with the medicine regime prescribed by the wearer's physician in addition to monitoring the potential side effects of medicine/supplements that the wearer takes while following the weight loss plan.

In various embodiments, the weight loss plan may include a sleep plan that is designed to support the wearer's weight loss goals. In some such embodiments, the sleep plan for the wearer may consist of at least one of: (1) a recommended amount of sleep; (2) a recommended time for the wearer to go to sleep; (3) a recommended time for the wearer to wake up; and/or (4) a recommended nap time. In various embodiments, the sleep plan may include a position for the wearer to sleep in, a temperature for the room where the wearer is sleeping, and/or a lighting condition for the room where the wearer is sleeping. In particular embodiments, the sleep plan may include advice for the wearer on avoiding certain types of light. In other embodiments, the sleep plan for the wearer may include suggestions for relaxation techniques. In various embodiments, the system may track the amount and quality of sleep the wearer is obtaining to determine if the wearer is complying with the sleep plan. The system may do this, for example, using one or more of the sensors (e.g., motion sensors) described herein. In some embodiments, the system may compare the actual daily sleep of the wearer to the prescribed sleep plan. In particular embodiments, the system may, in addition to tracking the wearer's sleep patterns, track the wearer's hormone levels (e.g., through perspiration), the wearer's glucose levels, and the wearer's activity levels.

At step 430C, at least partially in response to comparing the identity and the quantity of the food that the wearer is preparing to ingest to a predetermined weight loss plan, the system calculates, using at least one processor, one or more recommendations to assist the wearer in complying with the predetermined weight loss plan. In various embodiments, the system may analyze the wearer's calorie intake, compare the current calorie intake to the predetermined weight loss plan, analyze the wearer's current calories burned and calculate one or more activities that would allow the wearer meet the daily goals for the predetermined weight loss plan. In some embodiments, for example, the system may determine that the wearer is preparing to ingest a 500 calorie hamburger. In some such embodiments, the system may recommend that the wearer run for 45 minutes to offset the additional 500 calories.

In other embodiments, the system may determine that the wearer is preparing to ingest a food item that contains 1500 mg of sodium. If the predetermined weight loss plan contains a restriction on sodium, the system may recommend that the wearer not eat the food item since the food item contains too much sodium. In still other embodiments, the system may be configured to recommend an alternative food item that is substantially similar (e.g., the same, in the same category as the food item, etc.) as the food item that the wearer is preparing to ingest. In still other embodiments, the system may be configured to identify a particular food item, its associated nutritional information and then question the wearer's desire to ingest the particular food item. For example, the wearer may visit his local eating establishment and order "the heart attack" hamburger off the menu. Once the system identifies the food item and its associated nutritional information (e.g., calories, sodium content, fat content, etc.), the system may recommend to the wearer that he choose a more nutritional food item since "the heart attack" does not comply with the wearer's weight loss plan.

In various embodiments, the weight loss plan may also include allergy information associated with the wearer. For example, the wearer's predetermined weight loss plan may include information relating to the wearer's allergy to soy. Thus, once the system identifies that the wearer is ordering "the Heart Attack" hamburger, which based on the associated nutritional information indicates that it contains soy products, the system may send a recommendation to the wearer that states "the heart attack burger contains soy - you're allergic to soy - don't eat it!

In other embodiments, the system may be configured to monitor the wearer's exercise routine to determine whether the wearer is complying with daily exercise requirements. For example, the system may determine by mid-afternoon that that the wearer has only completed 50 percent of the steps required by the wearer's predetermined weight loss plan. In some such embodiments, the system may calculate additional exercise recommendations that the wearer may perform in order to meet the daily recommended steps set forth in the wearer's weight loss plan. In other embodiments, if the system detects that the wearer has been sitting for a large portion of the day, the system may calculate recommended activities that lessens the amount of time that the wearer is in the seated position.

In other embodiments, if the wearer's is engaging in a physical activity, the system may monitor whether the wearer is exercising at the proper intensity based on at least one of the wearer's movements, the wearer's heart rate, the wearer's respiration rate or the weight loss plan. If the system detects that the wearer is not exercising at a predetermined intensity established in the weight loss plan, the system may recommend that the wearer increase the pace of the wearer's movements.

In various embodiments, the system may monitor the wearer's compliance with the medicine regime and calculate recommendations to help the wearer comply with the medicine regime. For example, in some embodiments, the system may calculate reminders for the wearer to assist the wearer in taking medicine/supplements. In other embodiments, the system may identify a side effect of a medicine that the wearer is taking and recommend that the wearer monitor any weight gain since a common side effect of the medicine is weight gain.

In various embodiments, the system may be configured to monitor the wearer's compliance with the sleep plan to determine whether the wearer is complying with the sleep plan. For example, the system may use one or more sensors described herein (e.g., motion sensors) to determine by that the wearer has only slept a total of five hours for a particular day when the wearer's sleep plan called for seven hours per day. In some such embodiments, the system may calculate an additional nap time for the wearer in order to meet the daily recommended hours of sleep set forth in the wearer's weight loss plan. In other embodiments, if the system detects that the wearer has been sleeping for a large portion of the day, the system may calculate recommended activities that lessen the amount of time that the wearer is sleeping during the day. In still other embodiments, if the system determines that the wearer's sleep cycles are getting interrupted while the wearer is sleeping, for instance by sleep apnea, the system may recommend that wearer monitor any weight gain and comply with the wearer's dietary, exercise, and medicinal regimes since sleep apnea is a common side effect of gaining weight.

At Step 435C, the system notifies the wearer of one or more recommendations regarding the wearer's compliance with the predetermined weight loss plan. In various embodiments, the system notifies the wearer and/or a third-party of the one or more recommendations calculated by the system by sending a notification to the wearer's and/or the third-party's handheld computing devices. In particular embodiments, the system may notify the wearer and/or the third-party of the one or more recommendations by email or text message. In yet other embodiments, the system notifies the user of the one or more inconsistencies by communicating through a speaker coupled to the computerized eyewear.

In various embodiments, the system may notify the wearer and/or the third-party of a recommendation substantially immediately (e.g., immediately) after the system calculates the recommendation. In yet other embodiments, the system may notify the wearer and/or the third-party of all recommendations at the end of that day. In still other embodiments, the system may notify the wearer of an exercise recommendation when the wearer provides an indication to the system that the wearer is beginning to exercise.

In various embodiments, the third-party may be a relative or friend of the wearer so that the third-party may assist the wearer in meeting the requirements of the predetermined weight loss plan. In other embodiments, the third-party may be the wearer's nutritionist and/or physician to allow the nutritionist and/or physician to monitor the wearer's compliance with the predetermined weight loss plan. In still other embodiments, the third-party may be an insurance company that may adjust the wearer's life/health insurance rate(s) based on the wearer's compliance with the predetermined weight loss plan.

In various embodiments, the system, when executing the Behavior Pattern Analysis Module 400C, may omit particular steps, perform particular steps in an order other than the order presented above, or perform additional steps not discussed directly above.

### Health Monitoring Functionality

Various embodiments of a wearable health monitoring system are described below and may be implemented in any suitable context. Various aspects of the system's functionality may be executed by certain system modules, including a Health Data Acquisition Module 500, an Actionable Data Collection Module 600, a Decision Making Data Acquisition Module 700, and a Processing and Reporting Module 800. These modules are discussed more fully below.

### Health Data Acquisition Module

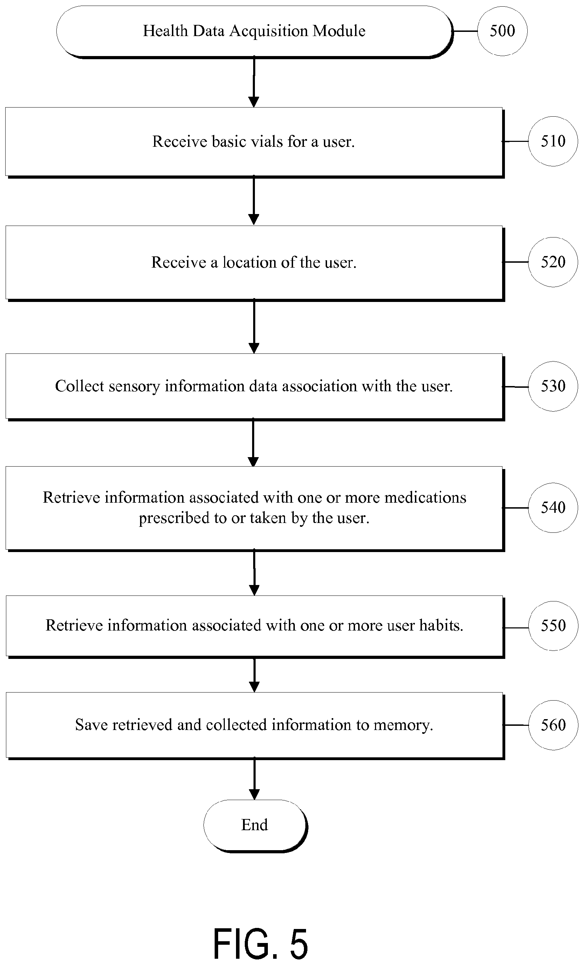

Referring to Figure 5, when executing the Health Data Acquisition Module5, the system begins, in various embodiments, at Step 510, by receiving basic vitals of a user. In particular embodiments, the system is configured to receive the basic vitals from one or more sensors associated with the wearable health monitoring device, such as any of the one or more sensors discussed above (e.g., such as a heart rate monitor, blood pressure sensor, etc.). In various embodiments, the system is configured to receive basic vitals such as, for example, blood pressure, heart rate, temperature, respiratory rate, blood oxygen levels, blood sugar levels, respiratory rate, or any other suitable vital.

The system continues, at Step 520, by receiving a location of the user. In various embodiments, the system is configured to receive the location of the user based at least in part on global position information determined from the wearable health monitoring device. In other embodiments, the system receives the location in response to the user providing his or her location to the system (e.g., via input on a mobile computing device such as a smartphone or tablet computer). In other embodiments, the system receives the location via voice input by the user, for example, via a microphone associated with the wearable health monitoring device, which may, for example, be configured to use suitable voice recognition and speech identification techniques to determine, based on the user's voice input, the location of the user.

Continuing at Step 530, the system collects sensory information associated with the user. The system may be configured to collect the sensory information via, for example, one or more cameras, one or more microphones, one or more accelerometers, or any other suitable sensory perceiving sensors. In particular embodiments, the sensory information may include information about what the user is touching, seeing, hearing, smelling, tasting, etc. In other embodiments, the sensory information may include information about the user's balance, acceleration, proprioception, nociception, interoception, and chronoception.

Next, the system, at Step 540, retrieves information associated with one or more medications that the user is taking or should be taking. In particular embodiments, the one or more medications the user should be taking may include one or medications prescribed by a health care professional. In particular embodiments, the system receives information associated with the one or medications the user is taking by receiving, from a camera associated with the wearable health monitoring device, one or more images of the one or more medications (e.g., or a container or bottle in which the one or more medications are stored) as the user is taking the one or more medications. The system may then use the image to determine the type of medication that the user is taking (by, for example, using OCR techniques to read alphanumeric information from a particular pill, and/or by determining the size, color, and shape of a pill, and searching a database of medications to determine the medication contained within the pill based on the pill's size, color, and/or shape, and/or by alphanumeric information printed on the pill.

In various embodiments, the system is configured to scan one or more machine-readable indicia (e.g., one or more bar codes, one or more QR codes, or any other suitable indicia) on a medicine bottle to determine what medication the user is taking. In other embodiments, the system receives the medication information from the user, for example via voice input or via input on a computing device such as a smartphone, or on the wearable health monitoring device itself.

In various embodiments, the system continues, at Step 550, by retrieving other information about one or more user habits. The one or more user habits may include dietary preferences (e.g., food the user eats), drug use, smoking, exercise frequency, sleep information, etc. The system may, in various embodiments, retrieve the information about the one or more user habits based in part on one or more images taken by an imaging device associated with the wearable health monitoring device (e.g., a front facing camera on a pair of glasses) as the user partakes in the one or more user habits while wearing the wearable health monitoring device.

In particular embodiments, the system continues, at Step 560, by saving any of the above data and/or information to memory. In various embodiments, the system is configured to save the information to local memory associated with the wearable health monitoring device. In other embodiments, the system may store the information remotely (e.g., in one or more remote servers or other suitable storage medium).

### Actionable Data Collection Module

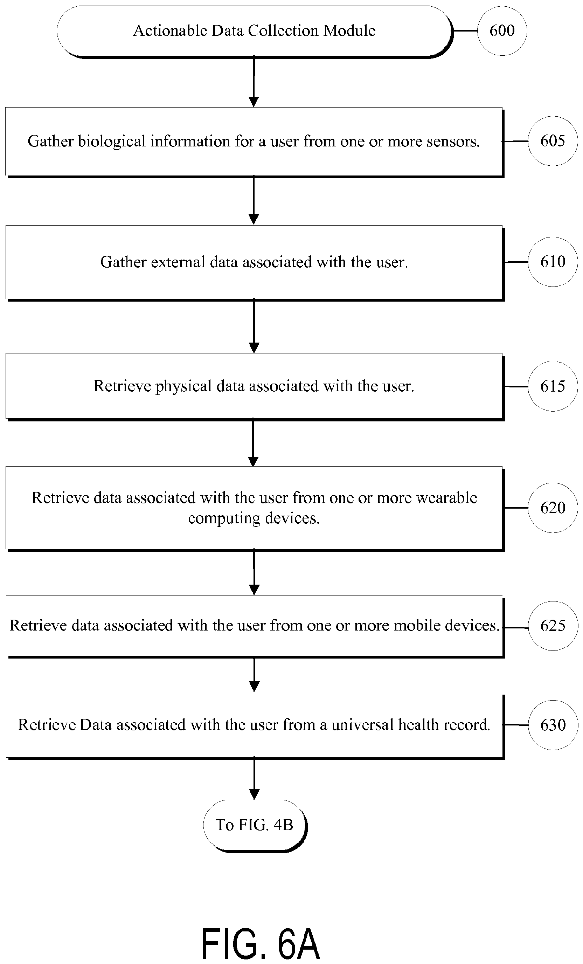

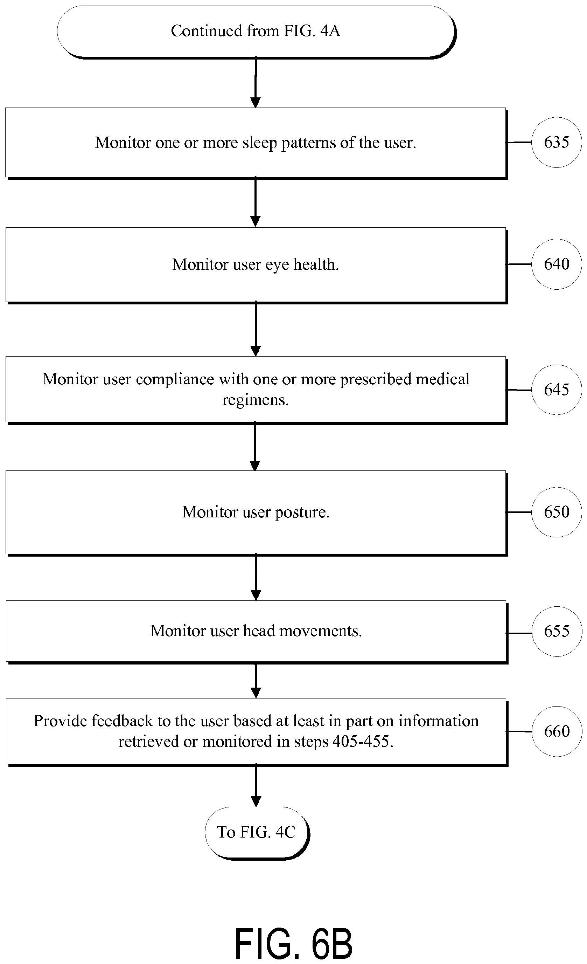

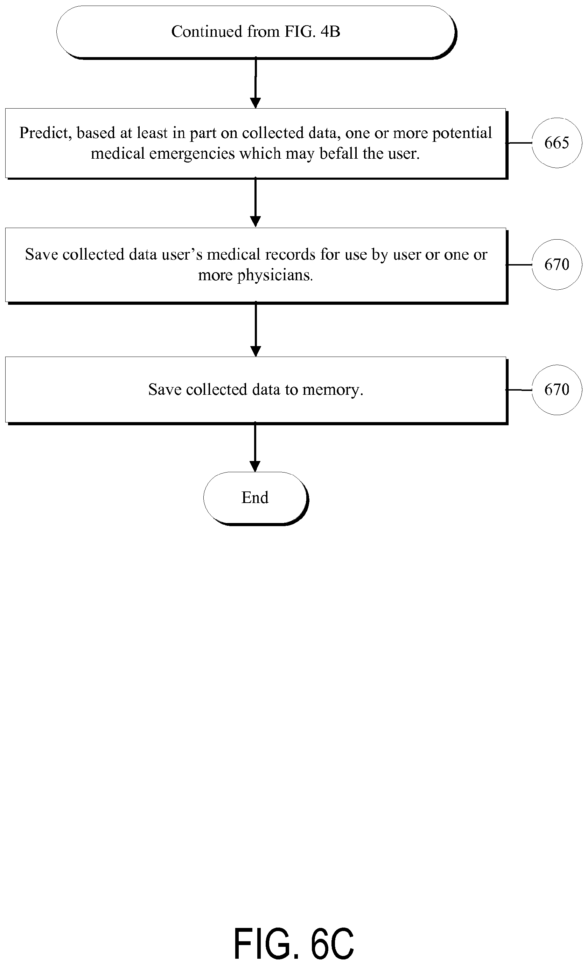

Referring to Figure 5A-6C, when executing the Actionable Data Collection Module 600, the system begins, in various embodiments, at Step 605, by gathering biological information about a user from one or more sensors. The biological information may include, for example, heart rate, temperature, geolocaiton, proximity to one or more other individuals or to a particular location (e.g., such as location proximate to a health-related outbreak or risk), humidity, accelerometer data, or other biological information associated with the user. In various embodiments, the system is configured to receive one or more biological samples from the user, such as, for example, blood, sweat, teardrops, etc. In particular embodiments, the system is configured to receive input (e.g., via a mobile computing device or voice input) describing one or more symptoms the user may be experiencing. In other embodiments, the system is configured to receive biological information based on one or more images of the user (e.g., taken from an imaging device associated with the wearable health monitoring system). The system may, for example, determine from the one or more images that the user looks discolored (e.g., has a face that is flushed or paler than normal).

The system continues, at Step 610, by gathering external data associated with the user. The external data may include, for example, calendar data (e.g., data associated with one or more places the user is travelling, data associated with one or more medical appointments, etc.). The user may, for example, have a vacation to a foreign country on their calendar, where the foreign country may require one or more vaccinations or have other health alerts that may be useful to the user. In other embodiments, the system is configured to gather external data such as one or more images of the user (e.g., from one or more social networking websites, from one or more email messages, etc.). In other embodiments, the system is configured to gather data from one or more e-mails sent to or received by the user. For example, the system may ascertain from an e-mail to the user's mother that the user is trying to have a baby or planning to train for a particular type of race or undertake any other action or activity that may warrant consultation with a physician or other medical professional.

The system continues, in particular embodiments, at Step 615 by retrieving physical data associated with the user. The physical data may include, for example, height, weight, build, body mass index (BMI), etc. In various embodiments, the system may retrieve the physical data, for example, by reading one or more scales as the user is standing on the scale (e.g., via an imaging device associated with the wearable health monitoring system), via input from the user or one or more health professionals measuring the user's physical data, or any other suitable technique. BMI may, for example, be determined based at least in part on the user's retrieved height, weight, age, and one or more other factors.

Continuing at Step 620, the system retrieves data associated with the user from one or more wearable computing devices, such as, for example, Nike+ FuelBand, FitBit, Jawbone UP, etc. The data retrieved from these devices may include, for example, activity level, number of steps taken, calories burned, or any other suitable data that such devices may track. Next, at Step 425, the system retrieves data from one or more mobile devices. The data may include, for example, data ascertained from one or more text messages (e.g., SMS messages) sent or received by the user, one or more applications used by or downloaded by the user, or any other suitable data from the one or more mobile devices.

In particular embodiments, the system continues at Step 630 by retrieving data associated with the user from a universal health record. The data may include, for example, information associated with the user's medical history, ailments for which the user has been treated, ailments for which the user is being treated, family health history information, susceptibility toward particular diseases associated with the user based on the users genealogy, or any other useful data available in a universal health record.

In various embodiments, at Step 635, the system is configured to monitor one or more sleep patterns of the user. The system may, for example, comprise one or more imaging devices directed toward the user's eyes, which may, for example, determine when the user is asleep or awake based at least in part on whether the user's eyes are open or closed. The system may utilize one or more accelerometers to determine the user's movement while sleeping, which may, for example, enable the system to determine when the user is experiencing REM (rapid eye movement) sleep. In various embodiments, the system may monitor, for example, hours of rest, hours spent in REM sleep, a time the user went to bed, a time the user got out of bed, napping behaviors, tossing and turning of the user during sleep, quality of sleep, sleep disturbances, sleep disorders (e.g., sleep apnea, bruxism, night terrors, insomnia, delayed sleep phase disorder, sleepwalking, etc.), elevation and position of the user's head during sleep, or any other suitable sleep related data.

In particular embodiments, the system continues, at Step 640, by monitoring user eye health. The system may, for example, monitor user eye inflammation, user vision (e.g., by determining whether a user often squints), or any other suitable eye health information, such as, for example information related to glaucoma, ankylosing spondylitis, high blood pressure, diabetes, sickle cell anemia, jaundice, colon polyps, heart disease, multiple sclerosis, leukemia, brain tumors, etc. The system may monitor eye health, for example, by collecting one or more tear and/or blood samples from the user, or using any other suitable technique described herein.

Next, at Step 645, the system monitors user compliance with one or more prescribed medical regimens. The system may, for example, monitor whether the user is taking particular prescribed medications using any suitable technique, such as one or more of the techniques described above. The system may be configured to monitor recommended diet and exercise regimens, by, for example, utilizing any of the data collected and/or retrieved by the system in the steps above, such as wearable computing device data or from data derived from one or more images taken from the imaging device associated with the wearable health monitoring device (e.g., such as by taking images of all food consumed by the user and using the images to determine what food and what quantities of the food the user has consumed).

At Step 650, the system monitors the user's posture, for example, by using one or more accelerometers, one or more gyroscopes, or any other suitable mechanism. Similarly, at Step 455, the system is configured to monitor head movements of the user. The system may monitor body position, for example, to monitor mental health. For example, a user suffering from symptoms of depression may exhibit different posture than a user that is not (e.g. different body language) in the form of slouching or other body language. In various embodiments, tracking of posture and head position may enable the system to ascertain potential spinal cord problems (e.g., scoliosis).

Continuing at Step 660, the system provides feedback to the user based at least in part on information retrieved or monitored in Steps 605 - 655. The system may, for example, recommend changes in behavior such as getting more sleep or eating healthier food, remind users to take one or more medications or vitamin supplements, recommend the user seek the advice of a physician before undertaking or continuing a particular activity, etc. The system may determine which feedback is appropriate by, for example, running one or more recommendation algorithms or scripts using the collected information. In a particular embodiment, one goal of providing the recommendations would be to improve a user's health or lifestyle.

At Step 665, the system continues by predicting, based at least in part on the collected data, one or more potential medical emergencies that may be likely to befall the user. The system may, for example, utilize any of the information collected above in combination with any other available information to predict and/or diagnose, for example, a heart attack, stroke, cataracts, macular degeneration, cancer, or any other medical emergency or illness that may befall the user. The system then, at Step 670, saves the collected data to the user's medical records for use by the user by one or more physicians or other medical professionals. Next, at Step 675, the system saves the collected data to memory. In various embodiments, the system is configured to save the information to local memory associated with the wearable health monitoring device. In other embodiments, the system may store the information remotely (e.g., in one or more remote servers or other suitable storage medium).

### Decision Making Data Acquisition Module

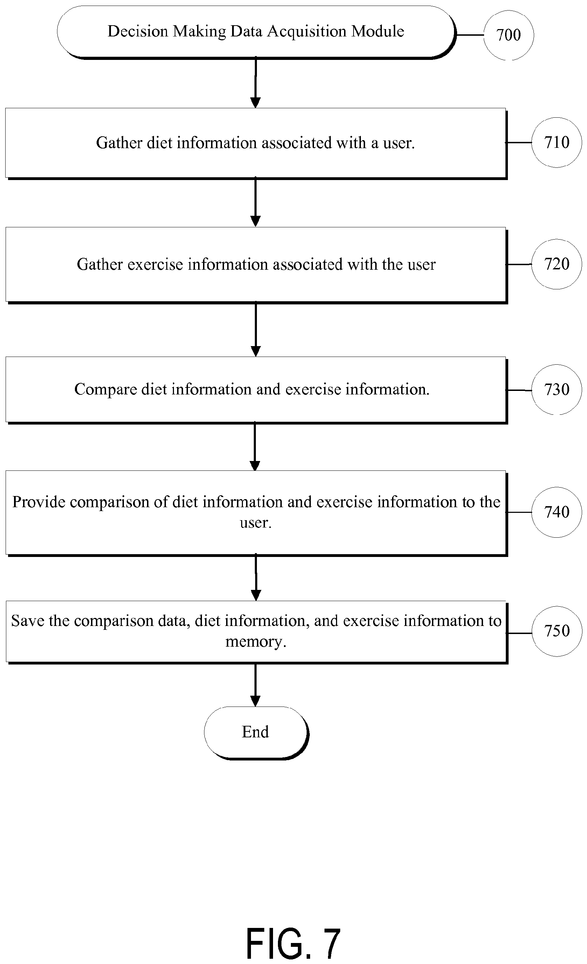

Referring to Figure 7, when executing the Decision Making Data Acquisition Module 700, the system begins, in various embodiments, at Step 710, by gathering diet information associated with the user. In particular embodiments, the system receives the diet information by receiving, from a camera associated with the wearable health monitoring device, one or more images of food that the user eats (e.g., as the user is preparing and/or consuming the food). In various embodiments, the system is configured to scan one or more machine-readable indicia (e.g., one or more bar codes, one or more QR codes, or any other suitable indicia) on food packaging for food that the user consumes. In other embodiments, the system receives the diet from the user, for example via voice input or via input on a computing device such as a smartphone, or on the wearable health monitoring device itself. In various embodiments, the diet information comprises food consumed as well as macronutrient information associated with the food (e.g., caloric content; grams of protein, fat, carbohydrates, etc.; sodium content; vitamin and mineral content; etc.).

Continuing at Step 720, the system continues by gathering exercise information associated with the user. In various embodiments, the exercise information comprises, for example, one or more particular exercises performed by the user, a number of repetitions of each exercise performed, an amount of weight lifted as part of each exercise, a distance travelled during one or more walks/runs/etc., a number of calories burned during a particular aerobic activity, an amount of time for which a particular activity is performed, etc. In particular embodiments, the system is configured to gather the exercise information from one or more images (e.g., and/or videos) of the user performing the activity, from one or more wearable computing device such as those discussed above, etc. In particular embodiments, the system is configured to gather the exercise information from input of the exercise information by the user.

The system continues, at Step 730, by comparing the diet information and exercise information. The system may for example, compare caloric intake via the user's diet with caloric expenditure via exercise from the exercise information. The system may, for example, predict a net weight loss and/or gain over a particular period of time based on the user's net caloric intake or output. The system may further compare diet and exercise information to determine risk of potential health issues such as heart disease, obesity, diabetes, etc. The system continues, at Step 740, by providing the comparison to the user. The system may, for example, provide information associated with progress toward a user's weight loss or weight gain goals. In a particular example, the system may provide the user with information that they may lose a particular amount of weight in a particular number of weeks if they maintain their current diet and exercise regimen.

Next, at Step 750, the system saves the collected data to memory. In various embodiments, the system is configured to save the information to local memory associated with the wearable health monitoring device. In other embodiments, the system may store the information remotely (e.g., in one or more remote servers or other suitable storage medium).

### Processing and Reporting Module

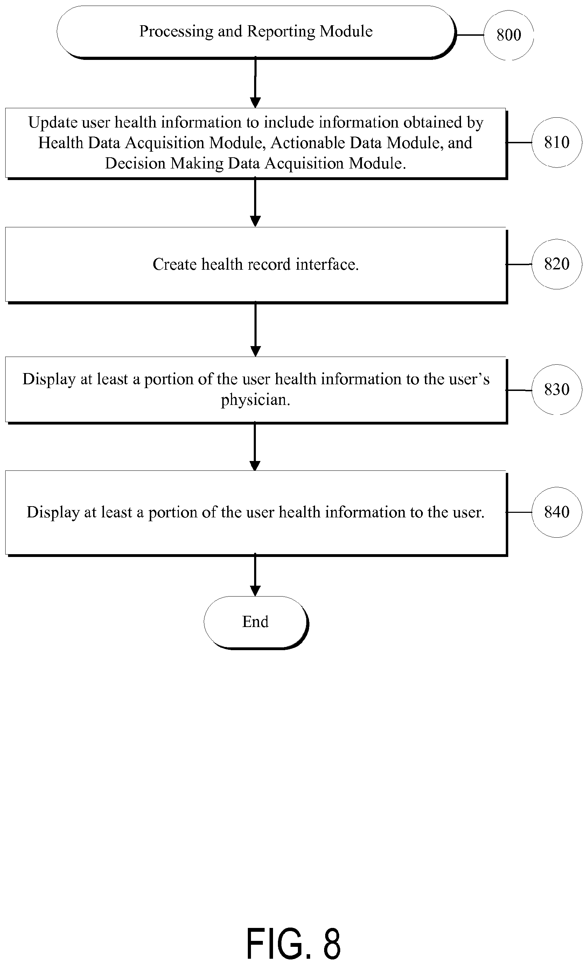

Referring to Figure 8, when executing the Processing and Reporting Module 800, the system begins, in various embodiments, at Step 810, by updating user health information to include information obtained via the Health Data Acquisition Module, Actionable Data Module, and Decision Making Data Acquisition Module described above. In particular embodiments, the user health information includes any suitable health related information and may be stored as part of any suitable record, in any suitable database, and/or on any suitable storage medium.

The system continues, at Step 820, by creating a health record interface, which may, for example, enable one or more users to access the user health information. The system then displays information to either the user's physician at Step 830 or the user at Step 840. The system may display the information in response to a request to display particular of the user's health information such as, for example, in the form of a user health report on sleep patterns, diet, exercise, medication, health history, blood pressure during a particular period, or any other suitable health information. In various embodiments, the system is further configured to provide user health information to one or more health insurance providers (which may, for example, provide discounts or have requirements about a user's diet and exercise choices), one or more life insurance providers (which may, for example, provide discounts for healthier lifestyles which can be tracked using the system), or any other party that may be interested in any of the information the system is capable of tracking.

### Exemplary User Experience For Behavior Pattern Analysis System

### Independent Living of Elderly

In a particular example of a user using the Behavior Pattern Analysis Module 300, the user may put on the wearable device in the morning and continue to wear the device throughout the day. In various embodiments, the wearable device may be operatively coupled (e.g., via a suitable wireless or wired connection) to a smart phone, a laptop, a desktop computer, an automated dialing apparatus, or any other computing device that can receive signals from the wearable device and either transmit the signals to a central system (e.g., via a wireless or wired telephone network) or analyze the signals and make decisions based on the received signals (e.g., call for help, notify a loved one, etc.). During this time, the system will track the movements of the user using the motion sensor, the accelerometer, the global positioning sensor, the gyroscope, and the front-facing camera. In this example, the user may be an elderly or infirm person that desires to live independently, but the person requires monitoring for events that deviate from the person's normal routine. Thus, by wearing the wearable device throughout the day, the device is able to track the user's movements and create certain patterns based on these movements for the user. The system may then store these patterns in a database while continuing to track the user's movements. Where the system detects that the user has deviated from the previously established pattern, the system may notify the user's physician, for example directly from the wearable device, or via the connected smartphone, computer or the automated dialing apparatus. Such deviations from the previously established pattern may include that the user falls, that the user wanders beyond preset boundaries (e.g., defined by one or more geofences), that the user begins sleeping longer than usual, that the user stops moving, or any other deviation from a previously established pattern of the user's normal routine.

For example, the user may be an Alzheimer's patient that has lucid moments and moments of memory loss. As has been established as a movement pattern by the wearable device, the patient takes a walk around the block every morning. However, if the patient wanders two blocks over and outside of the user's predetermined geo-fenced area, which is a deviation from the patient's normal pattern of movements, the system may alert the patient's caregiver of the inconsistency between the patient's current actions and the previously established patterns.

### Monitor Compliance with Medicine Regime

The system, in a particular example, will also monitor the user's compliance with a medicine regime. In order to establish the user's medicine regime pattern, the user may wear the wearable device to detect when the user takes medicine and what medicine is taken using the front-facing camera. The user may also speak the name of the medicine as the wearable device captures an image of the medicine the user is taking. The system is then able to establish a pattern of the user taking blood pressure medicine every morning after monitoring the user for a week. The system may then monitor the user's current medicine intake to compare the medicines the user takes and the time that the user takes the medicine to the previously established medicine regime pattern. If the user fails to take the blood pressure medicine on a particular morning, the system may notify the user, the user's caregiver, a health care provider, or a third party that the user has deviated from the previously established medicine regime.

### Exemplary User Experience For Gait Analysis

In a particular example, a pair of eyewear with embedded sensors may be used to monitor the user's gait over the course of one or more days (e.g., days, weeks, months, years, etc.). As the sensors measure the movements of the individual's body (e.g., the individual's head, legs, feet, etc...), the system may transmit the related movement data to a remote server where the information is stored in a suitable database. After receiving the data, a central server may process the data to identify one or more gait patterns for the individual. The system may then compare one or more of the individual's gait patterns with one or more known irregular gait patterns to determine whether the individual has an irregular gait pattern as discussed above.

The system may be utilized, for example, in the following construct. A patient may present to a physician complaining of weakness and decreased use of one leg. The physician may perform a routine physical, ask diagnostic questions, and have the patient walk briefly in order to physically demonstrate the purported condition. Upon observing the patient, the doctor may decide that the patient may potentially have a gait abnormality, but the physician cannot isolate the specific abnormality as presented by the patient. The physician may instruct the patient to wear the wearable gait monitoring device over the course of one or more days. During this time, the wearable gait monitoring device would obtain and record information regarding the individual's gait as discussed above.

The system may then use the information to identify one or more gait pattern irregularities as discussed above and generate a message to the user's treating physician indicating that the individual appears to have an abnormal gait. The system may optionally further display one or more potential medical conditions associated with that gait, e.g., amyotrophic lateral sclerosis, multiple sclerosis, etc. The physician may then meet with the individual to discuss the individual's condition, and/or to order additional testing to establish a particular diagnosis. For example, the physician may review the patient's medical history, presented gait pattern, and possible conditions contributing to the gait abnormality to diagnose and/or to order more tests to aid in the diagnosis of such medical conditions.

The system may similarly be used to analyze the fit of a particular prosthetic, or a user's recovery from an injury or surgery using similar techniques in combination with one or more of the methods described above.

### Exemplary User Experience Monitoring Compliance with a Weight Loss Plan

In a particular example of a wearer using the health monitoring device 156C (in the form of computerized eyewear) and the system 100C to monitor the wearer's compliance with a weight loss plan, the wearer may put on the computerized eyewear in the morning and continue to wear the device throughout the day. In various embodiments, the computerized eyewear may be operatively coupled (e.g., via a suitable wireless or wired connection) to a smart phone, a laptop, a desktop computer, or any other computing device that can receive signals from the computerized eyewear and either transmit the signals to a central system (e.g., via a wireless or wired telephone network, the Internet, etc.) or analyze the signals and make decisions based on the received signals (e.g., identify a food, a quantity of the food, calculate recommendations, etc.). During this time, the system (1) identifies a food item and a quantity of the food item that the wearer is preparing to ingest using the forward facing camera, the GPS unit, the microphone, etc., (2) tracks the food item and quantity, (3) compares the food item and quantity to a predetermined weight loss plan, (4) tracks the movements of the wearer using the motion sensor, the accelerometer, the global positioning sensor, the gyroscope, and the front-facing camera, (5) tracks the wearer's compliance with a medicine regime, as described in detail above, and/or (6) tracks the wearer's compliance with a sleep plan, as also described in detail above.

In this example, the wearer may be under doctor's orders to lose weight. Thus, by wearing the computerized eyewear throughout the day, the device is able to capture one or more images of one or more food items that the wearer is preparing to ingest, identify the one or more food items, and compare the nutritional information for the one or more food items to a predetermined weight loss plan set by the doctor. Continuing with this example, if the wearer goes out to eat, the system may receive a GPS signal that can be used to determine the location of the wearer. In addition to determining the location of the wearer, the system may also determine that the wearer is at a particular food establishment (e.g., a restaurant, a food store, etc.) located at the geographical location. Thus, based on the captured one or more images of the food item that the wearer is preparing to eat, the location of the wearer and the name of the food establishment, the system can identify the food item and the nutritional information associated with the food item by comparing the one or more captured images of the food item to images of food items available at the food establishment. Once the system determines a match and identifies the food item, the system may also identify nutritional information associated with the food item from a food information database accessed by the system.

In various embodiments, the system tracks the nutritional information associated with the food item and compares this information to the weight loss program. For example, the system may add the calories associated with the food item to a "total calories" consumed for the day value. In some embodiments, if the system determines that the food item will cause the wearer to exceed the total calories allocated to the wearer for the day under the weight loss program, the system may calculate one or more recommendations for the wearer. For example, the one or more recommendations may include a statement to the wearer that says "if you eat this food item, you must perform one or more of the following exercises for 40 minutes to offset the excess calories due to the food item." Another recommendation may be "Instead of eating the 'Blue Bomber hamburger' why not have the Chicken Caesar Salad?" In various embodiments, if the system determines that the wearer ingests the food item, the system may continue to remind the wearer that the wearer must perform additional physical activity to offset the excess calories consumed during the day.

### Exemplary User Experience For A Health Monitoring System

In a particular example of a user using the wearable health monitoring system, the system tracks the user through a typical day via a wearable health monitoring device embodied as a pair of eyewear. As may be understood from this disclosure, the system is configured to monitor the user's vitals, health, posture, diet, exercise and other factors throughout the day. When the user wakes up and puts the wearable health monitoring device on, the system takes baseline readings of the user's heart rate, for example via a touch sensor on the temple portion of the eyewear and, temperature, via a thermometer on the eyewear. The system may then gather data associated with the user's previous night's sleep such as, for example, length of sleep, REM sleep pattern based on eye movement captured via a camera on the eyewear that was positioned to face the user while the user was sleeping, and so on.

When the user is eating breakfast, the system captures data related to food and beverage intake. The system may, for example, ascertain that the user ate two fried eggs and a piece of toast for breakfast while drinking a glass of orange juice. The system may record information about the nutrition of the food and beverages consumed, for example, from a database of available information (e.g., the average number of calories per egg), or from the packaging in which the food was purchased. The user may then take a morning jog around their neighborhood, during which the eyewear would track the distance travelled by the user, the user's speed and/or elevation change, and duration of the user's jog. The eyewear may track this information via one or more accelerometers, or using GPS. The system may also continue to track the user's heart rate and body temperature during the run to monitor whether their heart rate, elevation and temperature change are normal for that type of activity.

After the run, on the user's commute to work, the system may track the time that the user is sedentary and the user's posture while the user is seated driving their vehicle. The device calculates the amount of time spent driving to/from work and other time spent sitting in the vehicle throughout the day. The system then utilizes a pedometer or accelerometer to track the number of steps taken by the user while the user is at work as well as time that the user spends seated at work. After recording and capturing what the user eats for lunch, the wearable health monitoring device reminds the user to take their medication, which the system confirms that the user takes in the proper dosage by using a camera to capture an image of the user taking his two pills.

The system, throughout the course of the user's day, also tracks the user's exposure, via one or more suitable sensors, to one or more portions of the electromagnetic spectrum. The system tracks UV exposure, for example, and may recommend that the user applies sunscreen during period of long exposure (e.g., when the user is outside touring a new facility at work). The system may warn the user, based on a change in the user's skin pigmentation detected by the camera, that the user has had too much sun exposure and should go inside or take other preventative measures to protect from the sun's rays.

The system may monitor the user's stress level (e.g., from their blood pressure, heart rate, etc.) and associate higher stress levels with particular co-workers with whom the user is interacting during periods of high stress or with particular projects on which the user is working during periods of high stress. The system may store this information to provide later to the user.

At the end of the day, the system may compile all information gathered throughout the day to provide the user with health updates such as stress prevention and coping techniques, progress toward the user's weight loss goals based on the user's food intake and exercise, etc. The system may further provide this information to one or more healthcare professionals who may be treating the user.

### Other Practical Implementations of the System

In particular embodiments, the system is configured to measure, gather, or retrieve any of the information discussed in this disclosure in response to any particular other information gathered by the system. The system may, for example, track blood sugar levels in response to detecting via a system camera that a user has eaten a particular food (e.g., for a diabetic patient). In another example, the system may track user eating habits in response to particular detected or measured stimuli such as, for example, stress, sleep deprivation, oversleep, etc. The system may be configured to determine any suitable associations between various measured data and other suitable data (e.g., other data measured by the system). For example, the system may measure change in blood pressure over time in response to determining that the user's activity level (e.g., frequency of exercise) has increased, or alternatively because the user's activity level has decreased.

### Conclusion

Many modifications and other embodiments of the invention will come to mind to one skilled in the art to which this invention pertains, having the benefit of the teaching presented in the foregoing descriptions and the associated drawings. Therefore, it is to be understood that the invention is limited to the scope of the appended claims. Although specific terms are employed herein, they are used in a generic and descriptive sense only and not for the purposes of limitation.

## Claims

1. A system for monitoring the gait of an individual to detect a medical condition associated with the individual, said system comprising:
a. a pair of glasses comprising one or more sensors for assessing the gait of the individual;
b. a computer system comprising a processor, the processor operatively coupled to a power source, the computer system being configured for:
c. receiving data from the one or more sensors
d. assessing, via a gait monitoring module, a gait of the individual based on the data from the one or more sensors
e. analyzing the assessed gait to determine the magnitude and phase of one or more relative peaks in a linear acceleration of the individual's head as the individual ambulates and based on the one or more relative peaks in linear acceleration during ambulation identify a gait abnormality and based on the gait abnormality determine whether the assessed gait includes one or more gait patterns that are consistent with a particular medical condition of an individual wearing the pair of glasses; and
f. in response to determining that the assessed gait includes the one or more gait patterns, generating an alert that communicates the particular medical condition.

2. The system of claim 1, wherein one of the one or more sensors is selected from a group consisting of a gyroscope, an accelerometer, a motion sensor, geomagnetic sensor, and an impact sensor.

3. The system of claim 1, wherein the step of assessing the gait of the individual based on the data from the one or more sensors comprises:
a. using the data from the one or more sensors to assess one or more movements of the individual's head as the individual ambulates; and
b. using the one or more movements of the individual's head to assess the gait of the individual.

4. The system of claim 1, wherein the step of analyzing the assessed gait to determine whether the assessed gait includes one or more gait patterns that are consistent with a particular medical condition comprises:
a. using the data to assess one or more movements of the individual's head as the individual ambulates;
b. comparing the one or more movements of the individual's head with one or more head movements that are associated with the particular medical condition; and
c. at least partially in response to determining that the one or more movements of the individual's head are at least similar to one or more head movements that are associated with the particular medical condition, determining that the assessed gait includes one or more particular gait patterns that are associated with the particular medical condition.

5. The system of claim 1, wherein the one or more gait patterns is foot drop.

6. The system of claim 5, wherein the particular medical condition is a medical condition selected from a group consisting of: stroke, amyotrophic lateral sclerosis, muscular dystrophy, Charcot Marie Tooth disease, multiple sclerosis, cerebral palsy, hereditary spastic paraplegia, and Friedrich's ataxia.

7. The system of claim 1, wherein the one or more gait patterns is propulsive gait.

8. The system of claim 7, wherein the particular medical condition is a medical condition selected from a group consisting of: carbon monoxide poisoning, manganese poisoning, and Parkinson's disease.

9. The system of claim 1, wherein the one or more gait patterns is waddling gait.

10. The system of claim 9, wherein the particular medical condition is a medical condition selected from a group consisting of: congenital hip dysplasia, muscular dystrophy, and spinal muscle atrophy.

11. The system of claim 1, wherein the step of analyzing the assessed gait to determine whether the assessed gait includes the one or more gait patterns that are associated with the particular medical condition comprises comparing one or more relative peaks in linear acceleration associated with the assessed gait of the individual with one or more relative peaks in linear acceleration of the one or more gait patterns that are associated with the particular medical condition.

12. The system of claim 1 further comprising:
a. analyzing the assessed gait to determine whether the assessed gait includes one or more gait patterns that are consistent with an improper fit of a prosthesis;
and
b. in response to determining that the assessed gait includes the one or more gait patterns, generating an alert that the prosthesis may fit the individual improperly.

13. The system of Claim 12, wherein the prosthesis is selected from a group consisting of a prosthetic limb and a prosthetic foot.

14. The system of claim 1, wherein the medical condition includes an indication of the level of an individual's recovery from a medical procedure.

15. The system of claim 1, wherein the medical condition includes an indication of the level of an individual's recovery from a particular injury.

## Patentansprüche

1. System zum Überwachen des Gangs eines Individuums, um eine medizinische Erkrankung zu detektieren, die mit dem Individuum verknüpft ist, wobei das System Folgendes umfasst:
a. eine Brille, die einen oder mehrere Sensoren zum Beurteilen des Gangs des Individuums umfasst;
b. ein Computersystem, das einen Prozessor umfasst, wobei der Prozessor operativ mit einer Stromquelle gekoppelt ist, wobei das Computersystem für Folgendes konfiguriert ist:
c. Empfangen von Daten von dem einen oder den mehreren Sensoren
d. Beurteilen, mittels eines Gangüberwachungsmoduls, eines Gangs des Individuums auf der Grundlage der Daten von dem einen oder den mehreren Sensoren
e. Analysieren des beurteilten Gangs, um die Magnitude und Phase einer oder mehrerer relativer Spitzen in einer linearen Beschleunigung des Kopfs des Individuums, während das Individuum geht, zu bestimmen, und auf der Grundlage der einen oder mehreren relativen Spitzen in der linearen Beschleunigung während des Gehens, Identifizieren einer Gangauffälligkeit, und auf der Grundlage der Gangauffälligkeit, Bestimmen, ob der beurteilte Gang ein oder mehrere Gangmuster beinhaltet, die mit einer bestimmten medizinischen Erkrankung des Individuums, das die Brille trägt, einhergehen; und
f. als Reaktion auf Bestimmen, dass der beurteilte Gang das eine oder die mehreren Gangmuster beinhaltet, Erzeugen eines Alarms, der die bestimmte medizinische Erkrankung kommuniziert.

2. System nach Anspruch 1, wobei einer des einen oder der mehreren Sensoren aus einer Gruppe ausgewählt ist, die aus einem Gyroskop, einem Beschleunigungsmesser, einem Bewegungssensor, geomagnetischen Sensor und einem Aufprallsensor besteht.

3. System nach Anspruch 1, wobei der Schritt zum Beurteilen des Gangs des Individuums auf der Grundlage der Daten von dem einen oder den mehreren Sensoren, Folgendes umfasst:
a. Verwenden der Daten von dem einen oder den mehreren Sensoren, um eine oder mehrere Bewegungen des Kopfs des Individuums, während das Individuum geht, zu beurteilen; und
b. Verwenden der einen oder mehreren Bewegungen des Kopfs des Individuums, um den Gang des Individuums zu beurteilen.

4. System nach Anspruch 1, wobei der Schritt zum Analysieren des beurteilten Gangs, um zu bestimmen, ob der beurteilte Gang ein oder mehrere Gangmuster beinhaltet, die mit einer bestimmten medizinischen Erkrankung einhergehen, Folgendes umfasst:
a. Verwenden der Daten, um eine oder mehrere Bewegungen des Kopfs des Individuums, während das Individuum geht, zu beurteilen;
b. Vergleichen der einen oder mehreren Bewegungen des Kopfs des Individuums mit einer oder mehreren Kopfbewegungen, die mit der bestimmten medizinischen Erkrankung verknüpft sind; und
c. mindestens teilweise als Reaktion auf Bestimmen, dass die eine oder mehreren Bewegungen des Kopfs des Individuums mindestens einer oder mehreren Kopfbewegungen ähnlich sind, die mit der bestimmten medizinischen Erkrankung verknüpft sind, Bestimmen, dass der beurteilte Gang ein oder mehrere bestimmte Gangmuster beinhaltet, die mit der bestimmten medizinischen Erkrankung verknüpft sind.

5. System nach Anspruch 1, wobei das eine oder die mehreren Gangmuster Fußheberschwäche sind.

6. System nach Anspruch 5, wobei die bestimmte medizinische Erkrankung eine medizinische Erkrankung ist, die aus einer Gruppe ausgewählt ist, bestehend aus: Schlaganfall, amyotropher Lateralsklerose, Muskeldystrophie, Charcot-Marie-Zahn-Krankheit, Multipler Sklerose, zerebraler Lähmung, hereditärer spastischer Paraplegie und Friedrich-Ataxie.

7. System nach Anspruch 1, wobei das eine oder die mehreren Gangmuster propulsiver Gang sind.

8. System nach Anspruch 7, wobei die bestimmte medizinische Erkrankung eine medizinische Erkrankung ist, die aus der Gruppe ausgewählt ist, bestehend aus: Kohlenmonoxidvergiftung, Manganvergiftung und Parkinson-Syndrom.

9. System nach Anspruch 1, wobei das eine oder die mehreren Gangmuster Watschelgang sind.

10. System nach Anspruch 9, wobei die bestimmte medizinische Erkrankung eine medizinische Erkrankung ist, die aus einer Gruppe ausgewählt ist, bestehend aus: angeborener Hüftdysplasie, Muskeldystrophie und spinaler Muskelatrophie.

11. System nach Anspruch 1, wobei der Schritt zum Analysieren des beurteilten Gangs, um zu bestimmen, ob der beurteilte Gang das eine oder die mehreren Gangmuster beinhaltet, die mit der bestimmten medizinischen Erkrankung verknüpft sind, Vergleichen einer oder mehrerer relativer Spitzen in linearer Beschleunigung, die mit dem beurteilten Gang des Individuums verknüpft ist, mit einer oder mehreren relativen Spitzen in linearer Beschleunigung des einen oder der mehreren Gangmuster umfasst, die mit der bestimmten medizinischen Erkrankung verknüpft sind.

12. System nach Anspruch 1, weiter umfassend:
a. Analysieren des beurteilten Gangs, um zu bestimmen, ob der beurteilte Gang ein oder mehrere Gangmuster beinhaltet, die mit einem ungeeigneten Sitz einer Prothese einhergehen; und
b. als Reaktion auf Bestimmen, dass der beurteilte Gang das eine oder die mehreren Gangmuster beinhaltet, Erzeugen eines Alarms, dass die Prothese dem Individuum nicht hinreichend passen könnte.

13. System nach Anspruch 12, wobei die Prothese aus einer Gruppe ausgewählt ist, die aus einer Beinprothese und einer Fußprothese besteht.

14. System nach Anspruch 1, wobei die medizinische Erkrankung eine Angabe des Genesungsgrads eines Individuums nach einer medizinischen Prozedur beinhaltet.

15. System nach Anspruch 1, wobei die medizinische Erkrankung eine Angabe des Genesungsgrads eines Individuums nach einer bestimmten Verletzung beinhaltet.

## Revendications

1. Système de surveillance de la démarche d'un individu afin de détecter un problème de santé associé à l'individu, ledit système comprenant :
a. une paire de lunettes comprenant un ou plusieurs capteurs pour évaluer la démarche de l'individu ;
b. un système informatique comprenant un processeur, le processeur étant couplé de manière fonctionnelle à une source d'alimentation, le système informatique étant configuré pour :
c. recevoir des données provenant des un ou plusieurs capteurs
d. évaluer, via un module de surveillance de démarche, une démarche de l'individu sur la base des données provenant des un ou plusieurs capteurs
e. analyser la démarche évaluée pour déterminer l'ampleur et la phase d'un ou de plusieurs pics relatifs d'accélération linéaire de la tête de l'individu lorsque l'individu déambule et, sur la base des un ou plusieurs pics relatifs d'accélération linéaire pendant la déambulation, identifier une anomalie de démarche et, sur la base de l'anomalie de démarche, déterminer si la démarche évaluée inclut un ou plusieurs schémas de démarche qui correspondent à un problème de santé particulier d'un individu portant la paire de lunettes ; et
f. en réponse à la détermination que la démarche évaluée inclut les un ou plusieurs schémas de démarche, générer une alerte qui communique le problème de santé particulier.

2. Système selon la revendication 1, dans lequel l'un des un ou plusieurs capteurs est sélectionné dans un groupe consistant en un gyroscope, un accéléromètre, un capteur de mouvement, un capteur géomagnétique, et un capteur de chocs.

3. Système selon la revendication 1, dans lequel l'étape d'évaluation de la démarche de l'individu sur la base des données provenant des un ou plusieurs capteurs comprend :
a. l'utilisation des données provenant des un ou plusieurs capteurs pour évaluer un ou plusieurs mouvements de la tête de l'individu lorsque l'individu déambule ; et
b. l'utilisation des un ou plusieurs mouvements de la tête de l'individu pour évaluer la démarche de l'individu.

4. Système selon la revendication 1, dans lequel l'étape d'analyse de la démarche évaluée pour déterminer si la démarche évaluée inclut un ou plusieurs schémas de démarche qui correspondent à un problème de santé particulier comprend :
a. l'utilisation des données pour évaluer un ou plusieurs mouvements de la tête de l'individu lorsque l'individu déambule ;
b. la comparaison des un ou plusieurs mouvements de la tête de l'individu avec un ou plusieurs mouvements de tête qui sont associés au problème de santé particulier ; et
c. au moins partiellement en réponse à la détermination que les un ou plusieurs mouvements de la tête de l'individu sont au moins similaires à un ou plusieurs mouvements de tête qui sont associés au problème de santé particulier, la détermination que la démarche évaluée inclut un ou plusieurs schémas de démarche particuliers qui sont associés au problème de santé particulier.

5. Système selon la revendication 1, dans lequel les un ou plusieurs schémas de démarche sont un pied tombant.

6. Système selon la revendication 5, dans lequel le problème de santé particulier est un problème de santé sélectionné dans un groupe consistant en : accident vasculaire cérébral, sclérose latérale amyotrophique, dystrophie musculaire, maladie de Charcot-Marie-Tooth, sclérose en plaques, paralysie cérébrale, paraplégie spastique héréditaire, et ataxie de Friedrich.

7. Système selon la revendication 1, dans lequel les un ou plusieurs schémas de démarche sont une démarche propulsive.

8. Système selon la revendication 7, dans lequel le problème de santé particulier est un problème de santé sélectionné dans un groupe consistant en : empoisonnement au monoxyde de carbone, empoisonnement au manganèse, et maladie de Parkinson.

9. Système selon la revendication 1, dans lequel les un ou plusieurs schémas de démarche sont une démarche dandinante.

10. Système selon la revendication 9, dans lequel le problème de santé particulier est un problème de santé sélectionné dans un groupe consistant en : dysplasie congénitale de la hanche, dystrophie musculaire, et atrophie musculaire spinale.

11. Système selon la revendication 1, dans lequel l'étape d'analyse de la démarche évaluée pour déterminer si la démarche évaluée inclut les un ou plusieurs schémas de démarche qui sont associés au problème de santé particulier comprend la comparaison d'un ou plusieurs pics relatifs d'accélération linéaire associés à la démarche évaluée de l'individu avec un ou plusieurs pics relatifs d'accélération linéaire des un ou plusieurs schémas de démarche qui sont associés au problème de santé particulier.

12. Système selon la revendication 1 comprenant en outre les étapes consistant à :
a. analyser la démarche évaluée pour déterminer si la démarche évaluée inclut un ou plusieurs schémas de démarche qui correspondent à une adaptation incorrecte d'une prothèse ; et
b. en réponse à la détermination que la démarche évaluée inclut les un ou plusieurs schémas de démarche, générer une alerte indiquant que la prothèse peut ne pas s'adapter correctement à l'individu.

13. Système selon la revendication 12, dans lequel la prothèse est sélectionnée dans un groupe consistant en un membre prothétique et un pied prothétique.

14. Système selon la revendication 1, dans lequel le problème de santé inclut une indication du niveau de rétablissement d'un individu après une procédure médicale.

15. Système selon la revendication 1, dans lequel le problème de santé inclut une indication du niveau de rétablissement d'un individu après une lésion particulière.
